(19) Europäisches Patentamt
European Patent Office
Office européen des brevets

(11) **EP 2 872 663 B1**

(12) **EUROPEAN PATENT SPECIFICATION**

(45) Date of publication and mention
of the grant of the patent:
**14.10.2020 Bulletin 2020/42**

(51) Int Cl.:
*A61L 31/02* (2006.01)          *A61L 31/08* (2006.01)
*A61L 31/14* (2006.01)

(21) Application number: **13816541.0**

(22) Date of filing: **10.07.2013**

(86) International application number:
**PCT/US2013/049970**

(87) International publication number:
**WO 2014/011803 (16.01.2014 Gazette 2014/03)**

(54) **BIODEGRADABLE ALLOY WIRE FOR MEDICAL DEVICES**

**BIOLOGISCH ABBAUBARER LEGIERUNGSDRAHT FÜR MEDIZINISCHE VORRICHTUNGEN**

**FIL EN ALLIAGE BIODÉGRADABLE POUR DES DISPOSITIFS MÉDICAUX**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.07.2012 US 201261669965 P**

(43) Date of publication of application:
**20.05.2015 Bulletin 2015/21**

(73) Proprietor: **Fort Wayne Metals Research Products Corporation**
**Fort Wayne, IN 46899 (US)**

(72) Inventor: **SCHAFFER, Jeremy E.**
**Leo, IN 46765 (US)**

(74) Representative: **Dr. Weitzel & Partner**
**Patent- und Rechtsanwälte mbB**
**Friedenstrasse 10**
**89522 Heidenheim (DE)**

(56) References cited:
**DE-C1- 4 242 757      US-A- 4 017 711**
**US-A1- 2007 156 231    US-A1- 2009 198 320**
**US-A1- 2009 198 320    US-A1- 2009 324 684**
**US-A1- 2010 174 367    US-A1- 2011 319 978**
**US-A1- 2011 319 978**

**Description**

BACKGROUND

1. Technical Field.

The present invention relates to biodegradable wire used in biomedical applications and, in particular, relates to wire alloys with controlled biodegradation for use in medical devices such as stents.

2. Description of the Related Art.

Stents are artificial tube-like structures that are deployed within a conduit or passage in the body to alleviate a flow restriction or constriction. Stents are commonly used in coronary arteries to alleviate blood flow restrictions resulting, e.g., from cardiovascular disease. However, stents may also be used in non-coronary vessels, the urinary tract and other areas of the body. Non-coronary applications range broadly from compliant pulmonary vessels of children with congenital heart disease (CHD), to atherosclerotic popliteal arteries of older patients with critical limb ischemia (CLI). Stented lesions may be long and tortuous as in the case of severe infrainguinal lesions, or short and relatively uniform as in mild pulmonary artery stenoses.

Examples of non-coronary stent applications include arteriovenous fistulas (AVFs) or false aneurysms, which may occur as a result of trauma due to gunshot wounds, falling accidents, or other blunt force incident. Such phenomena often occur in the upper limbs of the body where lack of perfusion can manifest as gangrene, severe pain, or local cyanosis. Critical limb ischemia associated with atherosclerosis can also result in the need for radial or axillary artery stenting, for example, to avoid amputation or other more serious morbidities. In contrast to most thoracoabdominal implantation sites (such as in coronary arteries), upper and lower limb anatomy is typically subjected to greater range of motion, thereby potentially increasing mechanical fatigue.

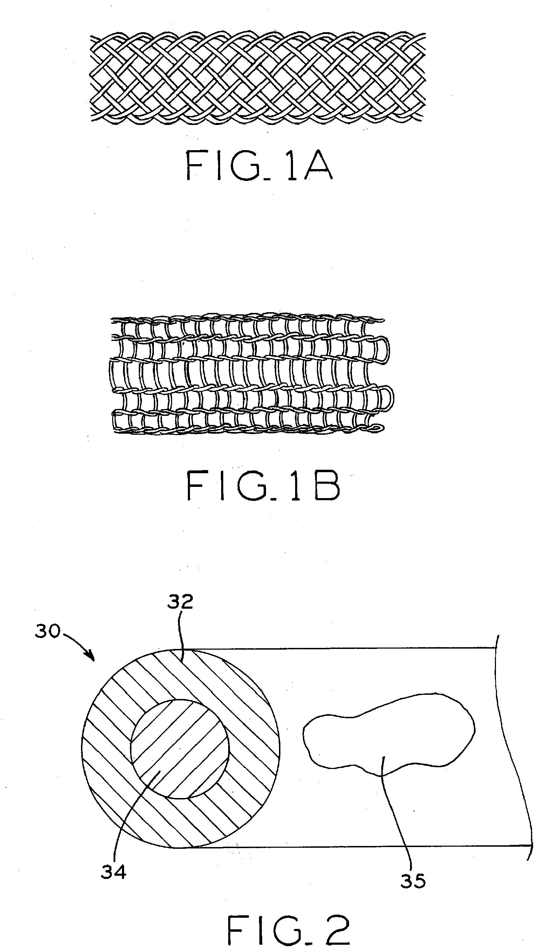

Typically, stents are made of either biocompatible metal wire(s) or polymeric fiber(s) which are formed into a generally cylindrical, woven or braided structure of the type shown in Figs. 1A and 1B. These types of stents are typically designed to be either "self-expanding", in which the stent may be made of a shape memory material, for example, and deploys automatically by expanding upon removal of a constricting force when released from a containment device, or "balloon-expanding", in which the stent is forcibly expanded from within by an inflatable balloon.

When a stent is implanted, it applies a radial force against the wall of the vessel in which it is implanted, which improves vessel patency and reduces acute closure or increases vessel diameter. In either case, the vessel usually achieves a new equilibrium by biological remodeling of the vessel wall over a period of weeks or months. After such remodeling is complete, the stent may no longer be needed for mechanical support and could potentially inhibit further natural positive remodeling of the vessel or limit re-intervention, for example. However, removal of an implanted stent may be difficult.

Many known stents are formed of corrosion-resistant and substantially non-biodegradable or non-bioresorbable metal materials which maintain their integrity in the body for many years after implantation. Design efforts for creating bioabsorbable stents have focused primarily on balloon-expandable technology for coronary pathologies, and may include polymer biodegradable stents using poly-L lactic acid (PLLA) and poly-L glycolic acid (PLGA), nutrient metals of magnesium (Mg), including alloys or powder metallurgy forms of magnesium, and iron (Fe), and iron-manganese alloys. Some research methods have also focused on hybrids including layered biodegradable polymers and bioabsorbable polymer coated nutrient metals. While such materials are resorbable, they may have low mechanical strength and resilience, and/or may confer inadequate control over the rate of bioabsorption (i.e., by biodegrading too slowly or quickly in vivo).

In the case of iron-manganese alloys such as Fe35Mn, a desirable dissolution rate may be achieved by the alloying of manganese with iron. For example, some iron-manganese alloys may be expected to degrade in vivo about twice as fast as a pure iron material. In the case of wire used for *in vivo* applications such as stents, a pure iron wire material may degrade over the course of about 2 years, while the iron-manganese material may degrade over the course of about 1 year. However, iron-manganese alloys may have insufficient elasticity and yield strength for some *in vivo* applications.

Low alloy steels, such as Fe-Mn or Fe-C, may exhibit uniform corrosion when the materials have no retained cold work. However, cold worked, wrought or otherwise mechanically conditioned Fe-Mn alloys (such as Fe35Mn) have potential for demonstrating stress corrosion cracking (SCC), which may lead to pitting type defects in the material surface. Once the pitting corrosion process gets underway, the ensuing non-uniform environmental attack on the wire material can potentially lead to a portion of the material separating from the main wire body because degradation progresses at a faster rate at the site of pitting corrosion as compared to the overall structure on either side of such site.

What is needed is a biodegradable metallic material and wire with sufficient mechanical properties and an appropriate degradation rate for use in biomedical applications, which represents an improvement over the foregoing.

US 2009/198320 A1 discloses a stent made of a biocorrodible iron alloy of formula Fe-Mn-X wherein the Mn content in the alloy is 5 to 30 wt% and X is one or more anti-corrosive elements selected from the group Pt, Pd, Ir, Rh, Re, Ru and Os with additional alloying of N in an amount of up to 0.8 wt% to control the degradation rate.

SUMMARY

The present invention is defined by a medical device with the features of claim 1 and a method of manufacturing a biodegradable wire with the steps of claim 9. The present disclosure provides a bioabsorbable wire material including manganese (Mn) and iron (Fe), in which one or more additional constituent materials (X) are added to control corrosion in an *in vivo* environment and, in particular, to prevent and/or substantially reduce the potential for pitting corrosion. The (X) element in the Fe-Mn-X system includes nitrogen (N), and optionally molybdenum (Mo) or chromium (Cr), or a combination of these. This promotes controlled degradation of the wire material, such that a high percentage loss of material, the overall material mass and volume may occur without fracture of the wire material into multiple wire fragments. The wire material has retained cold work for enhanced strength, for medical applications. In some applications, the wire material may be a fine wire suitable for use in resorbable *in vivo* structures such as stents.

In another exemplary application, the Fe-Mn-X material may be used as one or more constituents wire materials in a composite wire including, in cross-section, an outer shell or tube formed of a first biodegradable material and an inner core formed of a second biodegradable material. Both the shell and core may be adapted to resorb or disappear after post-operative vessel healing has occurred and vessel patency has been restored, or the shell may be the only resorbable component. Other materials suitable for use in the composite wire include nutrient-metal-composites and alloys of pure iron, manganese, magnesium, and zinc. Particular metals or metal alloys may be selected to provide a desired biodegradation rate and mechanical properties. The total rate of biodegradation of the wire, and therefore the duration of the overall mechanical integrity of the wire, may be controlled by the relative cross-sectional areas (i.e., the relative thicknesses) of the outer sheath and core material relative to the overall cross-sectional area of the wire.

When formed into a stent, for example, the first and second biodegradable materials of a composite may be different, and may have differing biodegradation rates. The first biodegradable material may degrade relatively slowly for retention of the mechanical integrity of the stent during vessel remodeling, and the second biodegradable material may degrade relatively quickly. The biodegradation rates may be inherently controlled, such as by selection of materials, and also may be mechanically controlled, such as by material thicknesses and the geometric configuration of the shell, core, or overall device.

The mechanical strength of the wire may be controlled to impart either a self-expanding character to a braided or knit stent device made from the wire, or may be controlled to provide a high strength wire for use in balloon-expandable wire-based stents. The mechanical strength and elastic resilience of the wire can be significantly impacted through thermomechanical processing.

In a form thereof, the present invention provides a method of manufacturing a wire, comprising the steps of: providing a wire made of Fe-Mn-X alloy, the wire comprising: iron (Fe) in the amount of at least 61 wt.%; manganese (Mn) in the amount of at least 31 wt.% manganese (Mn); and a quantity of an anti-corrosive alloying element (X) comprising at least nitrogen (N) in an amount between 0.01 wt.% and 0.45 wt.%, and strengthening the wire by imparting cold work at room temperature to the wire until a retained cold work ratio of grain length to grain width in the Fe-Mn-X alloy is between 10:1 and 50:1.

BRIEF DESCRIPTION OF THE DRAWINGS

The above mentioned and other features and objects of this invention, and the manner of attaining them, will become more apparent and the invention itself will be better understood by reference to the following description of embodiments of the invention taken in conjunction with the accompanying drawings, wherein:

Figs. 1A and 1B are perspective views of known stents;

Fig. 2 is a partial cross-sectional view of a composite wire made in accordance with the present disclosure;

Fig. 3a is a schematic view illustrating an exemplary forming process of monolithic wire using a lubricated drawing die;

Fig. 3b is a schematic view illustrating an exemplary forming process of composite wire using a lubricated drawing die;

Fig. 3c is an elevation view of a wire in accordance with the present disclosure, before a final cold working process;

Fig. 3d is an elevation view of the wire of Fig. 3c, after the final cold working process;

Fig. 4a is an elevation, cross-sectional view of a wire made from a solid, monolithic material $\alpha$ having diameter $D_W$ and radius $R_W$;

Fig. 4b is an elevation, cross-sectional view of a composite wire having diameter $D_W$ and including a core fiber made from a first material β and a shell surrounding the core fiber and made from a second material α, in which the thickness $T_1$ of the shell creates a surface area occupying 75% of the total cross-sectional area of the wire (β-25 v/v% α);

Fig. 4c is an elevation, cross-sectional view of a composite wire having diameter $D_W$ and including a core fiber made from a first material β and a shell surrounding the core fiber and made from a second material α, in which the thickness $T_1$ of the shell creates a surface area occupying 43% of the total cross-sectional area of the wire (β-57 v/v% α);

Fig. 4d is an elevation view illustrating the geometry of a braided stent having diameter $D_S$, the stent comprising 24 wire elements formed into a mesh tubular scaffold, in accordance with the present disclosure;

Fig. 5 is a picture of a braided stent structure formed from wire made in Example 1;

Fig. 6a is a graph illustrating tensile test results for sample materials used in Example 1, including engineering stress-strain plots for benchmark wire samples;

Fig. 6b is a graph illustrating tensile test results for sample materials used in Example 1, including engineering stress-strain plots for exemplary monolith wire samples;

Fig. 6c is a graph illustrating tensile test results for sample materials used in Example 1, including engineering stress-strain plots for exemplary bimetal wire samples;

Fig. 6d is a graph illustrating tensile test results for sample materials used in Example 1, including engineering stress-strain plots for additional exemplary bimetal wire samples;

Fig. 7a is a graph illustrating computed ultimate tensile strength for sample materials used in Example 1, in which error bars indicate one standard deviation;

Fig. 7b is a graph illustrating computed elongation and modulus of toughness for sample materials used in Example 1, in which error bars indicate one standard deviation;

Fig. 7c is a graph illustrating computed Young's modulus of elasticity for sample materials used in Example 1, in which error bars indicate one standard deviation;

Fig. 8a is a strain-life diagram for three monolithic materials which serve as benchmarks, with alternating strain defined as the difference between the maximum and mean strain (R = -1) plotted against the log of failure lifetimes (N) for samples tested at 60 Hz in ambient air having temperature = 23 ± 3°C;

Fig. 8b is a strain-life diagram similar to the diagram of Fig. 8a, illustrating test results for monolithic Fe with a 50% strain-hardening preparation, as compared with the 361L stainless steel benchmark;

Fig. 8c is a strain-life diagram similar to the diagram of Fig. 8a, illustrating test results for monolithic Fe with a 90% strain-hardening preparation, as compared with the 361L stainless steel benchmark;

Fig. 8d is a strain-life diagram similar to the diagram of Fig. 8a, illustrating test results for monolithic Fe with a 99% strain-hardening preparation, as compared with the 361L stainless steel benchmark;

Fig. 8e is a strain-life diagram similar to the diagram of Fig. 8a, illustrating test results for bimetal composite Fe-25Mg material, as compared with the 361L stainless steel benchmark;

Fig. 8f is a strain-life diagram similar to the diagram of Fig. 8a, illustrating test results for bimetal composite Fe -DFT-57Mg material, as compared with the 361L stainless steel benchmark;

Fig. 8g is a strain-life diagram similar to the diagram of Fig. 8a, illustrating test results for bimetal composite Fe35Mn-25MgZM21 material, as compared with the 361L stainless steel benchmark;

Fig. 9a is a graph illustrating material fractures plotted against a period of time, for a wire material made in accordance with the present disclosure and listed in Table 1-1 as trial no. 2;

Fig. 9b is a graph illustrating material fractures plotted against a period of time, for a wire material made in accordance with the present disclosure and listed in Table 1-1 as trial no. 5;

Fig. 9c is a graph illustrating material fractures plotted against a period of time, for a wire material made in accordance with the present disclosure and listed in Table 1-1 as trial no. 6;

Fig. 9d is a graph illustrating material fractures plotted against a period of time, for a wire material made in accordance with the present disclosure and listed in Table 1-1 as trial no. 8; and

Fig. 9e is a graph illustrating material fractures plotted against a period of time, for a wire material made in accordance with the present disclosure and listed in Table 1-1 as a control binary wire.

Corresponding reference characters indicate corresponding parts throughout the several views.

DETAILED DESCRIPTION

The present disclosure provides bioabsorbable wires which, when used to create a wire-based stent, produce dilatational force sufficient to promote arterial remodeling and patency, while also being capable of fully biodegrading over a specified period of time. This biodegradation may be controlled to protect against pitting corrosion, thereby minimizing or eliminating potential for embolization of the wire material in a protein environment. This controlled biodegradation promotes endothelial vasoreactivity, improved long term hemodynamics and wall shear stress conditions, enablement of reintervention and accommodation of somatic growth, and mitigates fracture risk over the long term.

Terminology

As used herein, "biodegradable," "bioabsorbable" and "bioresorbable" all refer to a material that is able to be chemically broken down in a physiological environment, *i.e.*, within the body or inside body tissue, such as by biological processes including resorption and absorption. This process of chemical breakdown will generally result in the complete degradation of the material and/or appliance within a period of weeks to months, such as 18 months or less, 24 months or less, or 36 months or less, for example. This rate stands in contrast to more "degradation-resistant" or permanent materials and/or appliances, such as those constructed from nickel-titanium alloys ("Ni-Ti") or stainless steel, which remain in the body, structurally intact, for a period exceeding at least 36 months and potentially throughout the lifespan of the recipient. Biodegradable metals used herein include nutrient metals, *i.e.*, metals such as iron, magnesium, manganese and alloys thereof, such as those including lithium. These nutrient metals and metal alloys have biological utility in mammalian bodies and are used by, or taken up in, biological pathways.

As used herein, "fatigue strength" refers to the load level at which the material meets or exceeds a given number of load cycles to failure. Herein, the load level is given as alternating strain, as is standard for displacement or strain-controlled fatigue testing, whereby terms are in agreement with those given in ASTM E606.

As used herein, a "load cycle" is one complete cycle wherein the unloaded (neutral) material is loaded in tension to a given alternating stress or strain level, unloaded, loaded again in compression to the same alternating stress or strain level, and returned to the neutral, externally unloaded position.

As used herein, "alternating strain" refers to the difference between the mean strain and the minimum strain level or the difference between the maximum strain and the mean strain in a strain-controlled fatigue cycle, where units are non-dimensional and given as percent engineering strain.

As used herein, "engineering strain" is given non-dimensionally as the quotient where the differential length associated with the load is the dividend and original length the divisor.

As used herein, "resilience" refers to an approximate quantification of the uniaxial elastic strain capability of a given wire test sample, and is calculated as the quotient of yield strength and modulus of elasticity, wherein yield strength is the dividend and modulus the divisor. Units: non-dimensional.

As used herein, "elastic modulus" is defined as Young's modulus of elasticity and is calculated from the linear portion of the tensile, monotonic, stress-strain load curve using linear extrapolation via least squares regression, in accordance with ASTM E111. Units are stress, in gigapascals (GPa).

As used herein, "yield strength" or "YS", in accordance with ASTM E8, refers to the 0.2% offset yield strength calculated from the stress-strain curve and gives quantitative indication of the point at which the material begins to plastically deform. Units are stress, in megapascals (MPa).

As used herein, "ultimate strength" or "UTS", in accordance with ASTM E8, refers to the maximum engineering stress required to overcome in order to rupture the material during uniaxial, monotonic load application. Units are stress, in mega-Pascals (MPa).

As used herein, "elongation" is the total amount of strain imparted to a wire during a uniaxial, monotonic tensile test, en route to specimen rupture, and is defined herein in accordance with ASTM E8. Units are non-dimensional, and are given as a percentage strain relative to the original specimen length.

As used herein, "energy to rupture" or "modulus of toughness" is defined herein as the amount of energy required to rupture a wire in a uniaxial tensile test. In a graphical stress-strain representation, the energy to rupture, as quantified herein, is the area under the curve for a given material. Units: millijoules per cubic millimeter ($mJ/mm^3$).

As used herein, "magnesium ZM21" refers to magnesium ZM21 alloy, otherwise known as ZM-21 or simply ZM21 alloy, which is a medium-strength forged magnesium alloy comprising 2 wt% Zn, 1 wt% Mn and a balance of Mg.

"Fe(II)" refers to iron ions of charge 2+ that may be associated with degradation products in a saline or bodily environment of iron or iron based alloys.

"Fe(III)" refers to iron ions of charge 3+ that may be associated with degradation products in a saline or bodily environment of Fe or Fe-based alloys.

"Mg(II)" refers to magnesium ions of charge 2+ that may be associated with degradation products in a saline or bodily environment of Mg or Mg-based alloys.

"RE" is used here to signify the rare earth elements given in the periodic table of elements and including elements such as Scandium, Yttrium, and the fifteen lanthanides, *i.e.* La, Ce, Pr, Nd, Pm, Sm, Eu, Gd, Tb, Dy, ..., to Lu.

"Nitinol" is a trade name for a shape memory alloy comprising approximately 50 atomic % Nickel and balance Titanium, also known as NiTi, commonly used in the medical device industry for highly elastic implants.

"DFT®" is a registered trademark of Fort Wayne Metals Research Products Corp. of Fort Wayne, IN, and refers to a bimetal or poly-metal composite wire product including two or more concentric layers of metals or alloys, typically at least one outer layer disposed over a core filament formed by drawing a tube or multiple tube layers over a solid metallic wire core element.

"Smooth muscle cells" (SMC) refer to mammalian cells of the smooth muscle that constitutes the vasotone-controlling muscle layer in, e.g., murine, porcine, human blood vasculature.

"OD" refers to the outside diameter of a metallic wire or outer shell.

"ID" refers to the inside diameter of a metallic outer shell.

Product Construction - Monolithic Wire

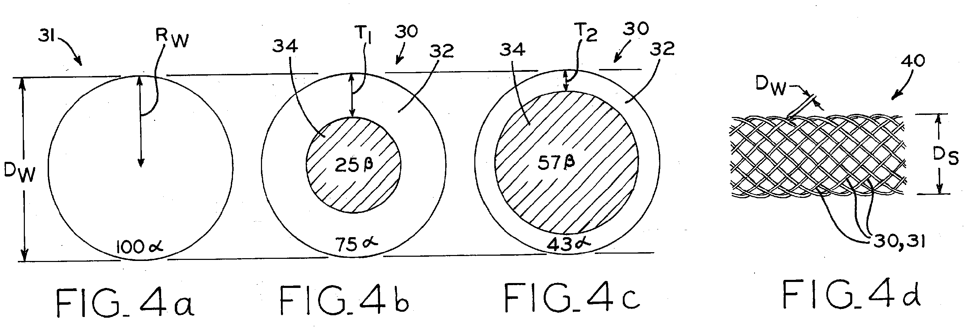

Material made in accordance with the present disclosure may be formed into wire products, such as fine-grade wire having an overall diameter $D_W$ (Figs. 4a-4d) of less than 1 mm. In one embodiment, a monolithic wire 31 (Fig. 4a) made of a biodegradable material in accordance with the present disclosure may have a uniform size and cross-sectional geometry along its axial length, such as the round cross-sectional shape having outer diameter $D_W$ as depicted. In another embodiment, a bimetallic composite wire 30 may be formed with separate core 34 and shell 32, as further described below.

Although round cross-sectional wire forms are shown in Figs. 4a-4d and described further below, it is contemplated that non-round wire forms may also be produced using the materials disclosed herein. For example, ribbon materials having rectangular cross-sectional shapes may be produced. Other exemplary forms include other polygonal cross-sectional shapes such as square cross-sectional shapes. Yet another exemplary wire form in accordance with the present disclosure includes hollow forms such as tubing, which may be used directly in an end product or as a shell in bimetallic composite wire as further described below.

In an exemplary embodiment, an Fe-Mn wire material is alloyed with N and optionally Cr or Mo or any combination thereof, which produces a wire material that is both amenable to cold-work processing, but also retains the uniform degradation properties associated with binary Fe-Mn wire materials. This wire material may be cold-worked into its final form as monolithic wire 31, as shown in Fig. 4a, or as bimetallic wire 30 as shown in Figs. 4b and 4c (further described below).

Fe-Mn wire material alloyed with N and optionally Cr or Mo or a combination thereof can therefore be cold-worked (by, e.g., drawing of the wire as described below) or otherwise wrought or mechanically conditioned to enhance the elasticity and/or yield strength of the material. Such enhancement of mechanical properties of the wire materials allows the wire to be tailored for use at a wider range of *in vivo* sites, such as in extremities where more extreme wire bends can be expected. At the same time, the alloy material exhibits uniform degradation and avoids pitting corrosion, thereby inhibiting separation of pieces of wire material from the larger body of wire.

The present Fe-Mn materials, alloyed with N and optionally Cr and/or Mo, provide at least two benefits to the alloy wire material which facilitate mechanical conditioning (e.g., drawing of wire materials) while maintaining uniform degradation. First, the alloy elements increase the point-of-zero-charge ("pH_pzc") to greater than pH 7 of surface oxide composition, which in turn increases serum protein adhesion and thereby protects the material from pitting corrosion *in vivo*. Second, the alloy elements increase the pitting resistance equivalent number (PREN, discussed in detail below), thereby guarding against stress corrosion cracking (SCC). The alloy elements may also increase material strength by means of solid solution strengthening.

As further discussed in the Examples section below, amounts of such alloying elements typically less than about 5 wt.% of the overall wire material are sufficient to achieve the aforementioned results. In certain exemplary embodiments, an alloy made in accordance with the present disclosure may comprise at least 61 wt.% Fe, at least 31 wt.% Mn, and balance N, or any combination with Cr or Mo. Materials in accordance with the present disclosure may include Fe in an amount as little as 45 wt.%, 55 wt.% or 63 wt.% and as much as 67 wt.%, 75 wt.% or 85 wt.%, or may include any amount of Fe within any range defined by any of the foregoing values. Materials in accordance with the present disclosure may include Mn in an amount as little as 15 wt.%, 25 wt.% or 33 wt.% and as much as 37 wt.%, 45 wt.% or 55 wt.%, or may include any amount of Mn within any range defined by any of the foregoing values.

In particular, the present material may comprise:

- A Fe-Mn-N alloy having between 0.05 wt.% and 0.45 wt.% Mo, such as: 65.0 wt.% Fe - 34.95 wt.% Mn - 0.05 wt.% N; 65.0 wt.% Fe - 34.85 wt.% Mn - 0.15 wt.% N; or 65.0 wt.% Fe - 34.55 wt.% Mn - 0.45 wt.% N. Nitrogen content of a Fe-Mn-N alloy, or of any Fe-Mn-X alloy, in accordance with the present disclosure may be may be as little as 0.01 wt.%, 0.02 wt.%, 0.03 wt.%, 0.04 wt.%, 0.05 wt.%, 0.06 wt.% or 0.07 wt.%, and as much as 0.10 wt.%, 0.11 wt.%, 0.12 wt.%, 0.13 wt.%, 0.14 wt.%, 0.15 wt.%, 0.25 wt.%, 0.35 wt.% or 0.45 wt.%, or may be any percentage within any range defined by any of the foregoing values.

- A Fe-Mn-Cr-Mo-N alloy having 35 wt.% Mn, 0.25 wt.% Cr, 0.50 wt.% Mo, 0.10 wt.% N and balance Fe.

Thus, the present Fe-Mn wire materials alloyed with N and optionally Cr, Mo or any combination thereof are appropriate for *in vivo* use after mechanical conditioning such as cold working. In addition, the wire products resulting from this final mechanical conditioning maintain their anti-ferromagnetic properties, consistent with the properties observed in Fe35Mn alloy systems, and therefore are compatible with application of magnetic resonance imagining MRI of a patient with an implanted medical device made with the present Fe-Mn alloy material. Bioabsorbability facilitated by the uniform surface erosion expected of Fe-Mn materials is also preserved.

Product Construction - Bimetallic Wire

A Fe-Mn alloy with a corrosion-control alloying element (e.g., N and opt. Cr and/or Mo) may also be used in the context of bimetallic wires, such that the ability to mechanically condition the material is preserved in one or both components of the bimetallic wire while also facilitating controlled biodegradation thereof.

Referring now to Fig. 2, bimetallic composite wire 30 has a circular cross section and extends along a longitudinal axis and includes outer shell, sheath, or tube 32 made of a first biodegradable material and a core 34 made of a second biodegradable material. Outer shell 32 may be formed as a uniform and continuous surface or jacket such as a tube with a generally annular cross-sectional shape, such that wire 30 may be coiled, braided, or stranded as desired.

As further described below, a first biodegradable material may be used for outer shell 32 while a second biodegradable material may be used for core 34. In an exemplary embodiment, one of the two biodegradable materials used for composite wire 30 is an iron-manganese alloy (Fe-Mn) including an additional constituent element (X) which protects against pitting corrosion, as described in detail above. This additional element (X) may include chromium (Cr), molybdenum (Mo), nitrogen (N) or any combination thereof.

The other of the two biodegradable materials may be any other material in accordance with the present disclosure. In one embodiment, the other material may be iron-based, such as pure metallic iron (Fe), an anti-ferromagnetic iron-manganese alloy (Fe-Mn) such as Fe-30Mn or Fe-35Mn, or another iron-based alloy (Fe alloy). In another embodiment, the other material of wire 30 may also be magnesium-based, such as pure magnesium (Mg) or a magnesium-based alloy (Mg alloy) such as ZM21 (Mg-2Zn-1Mn), AE21 (Mg-2A1-1RE, where RE is any of the Rare Earth metals such as Sc, Y, and the fifteen lanthanides, *i.e.*, La, Ce, Pr, Nd, Pm, Sm, Eu, Gd, Tb, Dy... to Lu), AE42 (Mg-4A1-2RE), WE43 (Mg-4Y-0.6Zr-3.4RE, as in yttrium, zirconium, RE).

For purposes of the present disclosure, exemplary bimetal composite wires (such as those discussed in detail in the Examples section below) are expressed as a first material and a second material comprising a specified balance percentage of the total wire cross-sectional area. Thus, the expression may read [First material]-DFT-X%[Second material], where the second material is X% of the cross-sectional area and the first material is the balance of the wire cross-section, i.e., (100-X)%. For example, Fe35Mn-DFT-25%Mg is 25% Mg of the total cross sectional area of wire 30, with the remaining 75% of the cross-sectional area occupied by Fe35Mn. One embodiment of this construct is illustrated in Fig. 4b, where core 34 made of $\beta$ material is Mg and shell 32 made of $\alpha$ material is Fe35Mn.

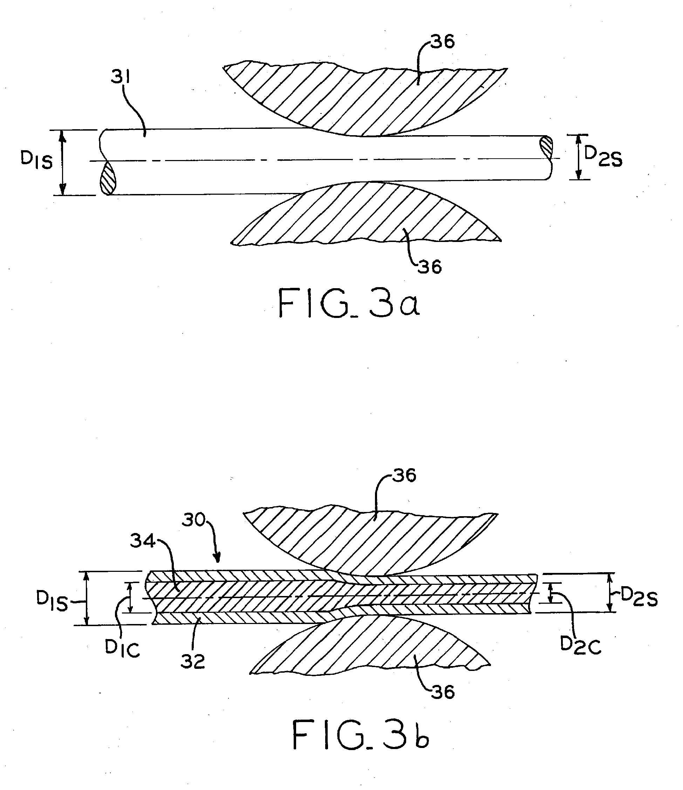

As shown in Figs. 4a-4c, the relative proportions of metals used in the experiments can be varied by varying the relative thicknesses of monolithic wire 31, or of core 34 and shell 32 of composite wire 30. Fig. 4a shows a monolithic wire material 31 made entirely of a first material $\alpha$ having outer cross-sectional diameter $D_W$. Fig. 4b shows a wire 30, such as a wire for a stent, in which shell 32 is made of a first material $\alpha$ having a thickness $T_1$, which is sufficient relative to overall diameter $D_W$ to ensure that material $\alpha$ occupies 75% of the total cross-sectional area of wire 30, while core 34 is formed from a second material $\beta$ occupies the balance (25%) of the cross-sectional area of wire 30. Fig. 4c shows a wire 30, such as a wire for a stent, in which shell 32 is made of a first material $\alpha$ having reduced thickness $T_2$ such that material $\alpha$ occupies 43% of the total cross-sectional area of wire 30, while core 34 formed from second material $\beta$ occupies the balance (57%) of the cross-sectional area of wire 30. Where wires 31 or 30 are drawn, diameter $D_W$ is the same as finished diameter $D_{2S}$ (Figs. 3a and 3b) of the drawn wire material.

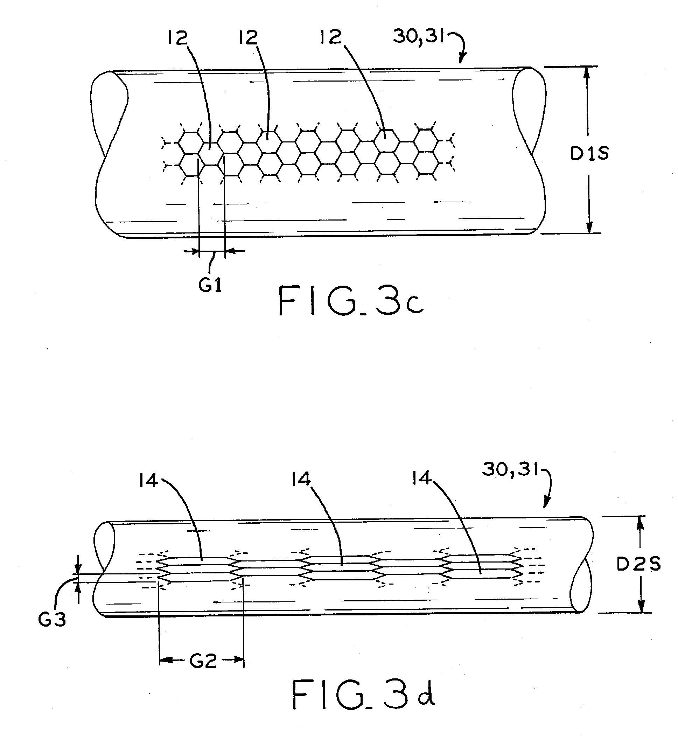

In some exemplary embodiments, outer diameter $D_W$ is 125 $\mu$m. Stent 40, made from wire 30 and/or 31, has total outside diameter $D_S$, which may be about 7 mm, for example. Stent 40 may be a tubular mesh stent scaffold manufactured from wires 30, 31, or a combination of wires 30 and 31. An exemplary strut thickness (i.e., wire diameter $D_W$) of 127 $\mu$m and expanded tubular diameter $D_S$ of 7 mm, as per Figure 3(d), are selected as dimensions similar to current self-expanding stent designs which are used in peripheral vessel scaffolding.

For composite wires 30 incorporated into stents or other *in vivo* structures, the first (i.e., outer) biodegradable material may be chosen to degrade *in vivo* at a slower rate than the second (i.e., inner) biodegradable material, such that overall structural integrity and strength are substantially maintained for a period of time after initial implantation while the slower-degrading outer material bioabsorbs or bioresorbs. This initial period is followed by relatively rapid biodegradation as the outer material erodes, exposing the inner material to biodegradation by interaction with substances in the *in vivo* environment. In certain exemplary embodiments, this construction modality is employed where approximately equal amounts of the first and second biodegradable materials are used. However, as discussed below, the relative degradation rates may be varied by providing wire constructs having varying amounts of the first and second biodegradable materials.

It is contemplated that outer shell 32 and core 34 may be formed from the same material or different materials, and that either shell 32 or core 34 may be formed from any of the above-mentioned materials as required or desired for a particular application. For example, shell 32 may be formed of a relatively slower-biodegrading material and core 34 may be formed of a relatively faster-biodegrading material. In other embodiments, this arrangement may be reversed, wherein shell 32 may be formed of a relatively faster-biodegrading material and core 34 may be formed of a relatively slower-biodegrading material. Moreover, stents made from wire produced in accordance with the present disclosure provide well-designed control over the mechanics and page of the overall degradation rate of the constituent wires (and therefore, also of the stent structure itself), thereby facilitating therapeutic optimization.

In one exemplary embodiment, composite wires 30 designed for *in vivo* applications may have shell 32 made from the above-mentioned Fe-Mn-X alloy which prevents pitting corrosion along the outer surface thereof, and may have core 34 formed from Mg or an Mg-based alloy. This Mg or Mg-based core 34 is a relatively softer material as compared to the Fe-Mn-X shell 32, such that core 34 provides mechanical stress relief to shell 32 and thereby prevents fracture of shell 32 during formation and use of wire 30. For example, exemplary wires 30 may include an Fe-Mn-X composition for shell 32, core 34, or both shell 32 and core 34. These Fe-Mn-X compositions may be selected from any of the material compositions described herein, including the compositions described above with respect to monolithic wire embodiments. For example, wire 30 may be formed of the following material compositions:

- FeMnX-DFT-25%Mg: 75% iron-manganese-X, where Fe and Mn are provided in quantities in accordance any of the material compositions described herein and X is an anti-pitting corrosion alloying element with one or more of N, Cr or Mo in accordance any of the material compositions described herein, and 25% magnesium; and
- FeMnX-DFT-57%Mg: 43% iron-manganese-X, where Fe and Mn are provided in quantities in accordance any of the material compositions described herein and X is an anti-pitting corrosion alloying element with one or more of N, Cr or Mo in accordance any of the material compositions described herein, and 57% magnesium;
- FeMnX-DFT-25%MgZM21: 75% iron-manganese-X, where Fe and Mn are provided in quantities in accordance any of the material compositions described herein and X is an anti-pitting corrosion alloying element with one or more of N, Cr or Mo in accordance any of the material compositions described herein, and 25% magnesium ZM21. This alloy may also be referred to in the present disclosure as FeMnX-DFT-25Mg, FeMnX-25MgZM21, or FeMnX-25Mg;

Shell 32 of the wire may be partially or fully coated with a biodegradable polymer 35 (Fig. 2) that may be drug-eluting to further inhibit neointimal proliferation and/or restenosis. Suitable biodegradable polymers include poly-L lactic acid (PLLA) and poly-L glycolic acid (PLGA), for example. The wire may be coated either before, or after being formed into a medical device such as stent 40 (Fig. 4d).

## Wire Production

An alloy in accordance with the present disclosure is first formed in bulk, such by casting an ingot, continuous casting, or extrusion of the desired material. This bulk material is then formed into a suitable pre-form material (e.g., a rod, plate or hollow tube) by hot-working the bulk material into the desired pre-form size and shape. For purposes of the present disclosure, hot working is accomplished by heating the material to an elevated temperature above room temperature and performing desired shaping and forming operations while the material is maintained at the elevated temperature. The resulting pre-form material, such an ingot, is then further processed into a final form, such as a rod, wire, tube, sheet or plate product by repetitive cold-forming and annealing cycles. In one exemplary embodiment, this further processing is used to fabricate wires 30 and/or 31, as further described below.

Monolithic wire 31 may be initially produced using conventional methods, including a schedule of drawing and annealing in order to convert the pre-form material (such as an ingot or rod) into a wire of a desired diameter prior to final processing. That is, the pre-form material is drawn through a die 36 (Fig. 3a) to reduce the outer diameter of the ingot slightly while also elongating the material, after which the material is annealed to relieve the internal stresses (i.e., retained cold work) imparted to the material by the drawing process. This annealed material is then drawn through a new die 36 with a smaller finish diameter to further reduce the diameter of the material, and to further elongate the material. Further annealing and drawing of the material is iteratively repeated until the material is formed into a wire construct ready for final processing into wire 31.

To form wire 30 (Fig. 2), core 34 is inserted within shell 32 to form an initial wire construct, and an end of the wire construct is then tapered to facilitate placement of the end into a drawing die 36 (Fig. 3b). The end protruding through the drawing die 36 is then gripped and pulled through the die 36 to reduce the diameter of the construct and bring the inner surface of shell 32 into firm physical contact with the outer surface of core 34. More particularly, the initial drawing process reduces the inner diameter of shell 32, such that shell 32 closes upon the outer diameter of core 34 such that the inner diameter of shell 32 will equal the outer diameter of core 34 whereby, when viewed in section, the inner core 34 will completely fill the outer shell 32 as shown in Fig. 2.

The step of drawing subjects wire 30 or 31 to cold work. For purposes of the present disclosure, cold-working methods effect material deformation at or near room temperature, e.g. 20-30 °C. In the case of composite wire 30, drawing imparts cold work to the material of both shell 32 and core 34, with concomitant reduction in the cross-sectional area of both materials. The total cold work imparted to wire 30 or 31 during a drawing step can be characterized by the following formula (I):

$$ cw = 1 - \left( \frac{D_2}{D_1} \right)^2 \times 100\% \qquad (I) $$

wherein "cw" is cold work defined by reduction of the original material area, "$D_{2S}$" is the outer cross-sectional diameter of the wire after the draw or draws, and "$D_{1S}$" is the outer cross-sectional diameter of the wire prior to the same draw or draws.

Referring to Figs. 3a and 3b, the cold work step may be performed by the illustrated drawing process. As shown, wire 30 or 31 is drawn through a lubricated die 36 having an output diameter $D_{2S}$, which is less than diameter $D_{1S}$ of wire 30 or 31 prior to the drawing step. The outer diameter of wire 30 or 31 is accordingly reduced from pre-drawing diameter $D_{1S}$ to drawn diameter $D_{2S}$, thereby imparting cold work cw.

Alternatively, net cold work may be accumulated in wire 30 or 31 by other processes such as cold-swaging, rolling the wire (e.g., into a flat ribbon or into other shapes), extrusion, bending, flowforming, or pilgering. Cold work may also be imparted by any combination of techniques including the techniques described here, for example, cold-swaging followed by drawing through a lubricated die finished by cold rolling into a ribbon or sheet form or other shaped wire forms. In one exemplary embodiment, the cold work step by which the diameter of wire 30 is reduced from $D_{1S}$ to $D_{2S}$ is performed in a single draw and, in another embodiment, the cold work step by which the diameter of wire 30 is reduced from $D_{1S}$ to $D_{2S}$ is performed in multiple draws which are performed sequentially without any annealing step therebetween.

For processes where drawing process is repeated without an intervening anneal on composite wire 30, each subsequent drawing step further reduces the cross section of wire 30 proportionately, such that the ratio of the sectional area of shell 32 and core 34 to the overall sectional area of wire 30 is nominally preserved as the overall sectional area of wire 30 is reduced. Referring to Fig. 3b, the ratio of pre-drawing core outer diameter $D_{1C}$ to pre-drawings shell outer diameter $D_{1S}$ is the same as the corresponding ratio post-drawing. Stated another way, $D_{1C}/D_{1S} = D_{2C}/D_{2S}$.

Thermal stress relieving, otherwise known in the art as annealing, at a nominal temperature not exceeding the melting point of either the first or second materials, is used to improve the ductility of the fully dense composite between drawing steps, thereby allowing further plastic deformation by subsequent drawing steps. Further details regarding wire drawing are discussed in U.S. Patent Application Serial No. 12/395,090, filed February 27, 2009, published as US20090260852, entitled "Alternating Core Composite Wire", assigned to the assignee of the present invention. When calculating cold work cw using formula (I) above, it is assumed that no anneal has been performed subsequent to the process of imparting cold work to the material. Heating wire 30 to a temperature sufficient to cause recrystallization of grains eliminates accumulated cold work, effectively resetting cold work cw to zero.

On the other hand, wires 30 or 31 subject to drawing or other mechanical processing without a subsequent annealing process retain an amount of cold work. The amount of retained work depends upon the overall reduction in diameter from $D_{1S}$ to $D_{2S}$, and may be quantified on the basis of individual grain deformation within the material as a result of the cold work imparted. Referring to Fig. 3c, wire 31 is shown in a post-annealing state, with grains 12 shown substantially equiaxed, i.e., grains 12 define generally spheroid shapes in which a measurement of the overall length G1 of grain 12 is the same regardless of the direction of measurement. After drawing wire 31 (as described above), equiaxed grains 12 are converted into elongated grains 14 (Fig. 3d), such that grains 14 are longitudinal structures defining an elongated grain length G2 (i.e., the longest dimension defined by grain 14) and a grain width G3 (i.e., the shortest dimension defined by grain 14). The elongation of grains 14 results from the cold working process, with the longitudinal axis of grains 14 generally aligned with the direction of drawing, as illustrated in Fig. 3d.

The retained cold work of wire 31 after drawing can be expressed as the ratio of the elongated grain length G2 to the width G3, such that a larger ratio implies a grain which has been "stretched" farther and therefore implies a greater amount of retained cold work. By contrast, annealing wire 31 after an intermediate drawing process recrystallizes the material, converting elongated grains 14 back to equiaxed grains 12 and "resetting" the retained cold work ratio to 1:1.

As noted above, monolithic biodegradable wire 31 formed of the present Fe-Mn-X class of alloys may be subject to the cold work processing described herein. These materials may have the ability to undergo - and may in fact be subject to - at least 85% cold work, and in some cases up to 99.99% cold work, thereby enabling a wide range of cold work strengthening options. Cold work may be imparted as a finishing step, as discussed above, such that the "retained cold work" ratio of grain length G2 to grain width G3 is as little as 10:1, 15:1, 20:1 or 25:1, and as much as 30:1, 35:1, 40:1, 45:1 or 50:1, or may be any ratio within any range defined by any of the foregoing values. Yield strengths YS may be in excess of 1000 MPa, and in some cases more than 1700 MPa. Ultimate tensile strength UTS is about 1380 MPa, and in some cases more than 2070 MPa.

Other exemplary monolithic biodegradable wires, discussed in detail in Example 3 below, may also include the following materials: iron with 50% retained cold work (which may also be referred to in the present disclosure as Fe 50, Fe-50 or Fe-50CW); iron with 90% retained cold work (which may also be referred to in the present disclosure as Fe 90, Fe-90 or Fe-90CW); and iron with 99% retained cold work (which may also be referred to in the present disclosure as Fe 99, Fe-99 or Fe-99CW).

Annealing processes may also be employed in wires with retained cold work, in which the annealing temperatures and/or durations are kept low enough to "soften" the materials while also preventing recrystallization of the material. For composite wire 30, the softening point of the constituent materials is controlled by introducing cold work into the composite structure after joining the metals as described above. For either monolithic wire 31 or composite wire 30, deformation energy is stored in the cold-worked structure, and this energy serves to reduce the amount of thermal energy required for stress relief of the wire material. This processing facilitates annealing of the composite structure at temperatures in the range of 40% to 50% of the melting point of the material, such that a low-temperature annealing process provides ductility to the metal wire material without converting elongated grains 14 back to equiaxed grains 12. Such ductility facilitates spooling of the wire, as discussed below, and renders the wire suitable for *in vivo* uses where low ductility would be undesirable.

After production, the resulting wire 30 may then be braided into the shape of a stent such as that of Fig. 1A, knitted into the shape of a stent such as that of Fig. 1B, or otherwise formed into a medical device such as a vascular or gastric stent, aneurysm clotting device, or blood filter, for example. For the foregoing applications, wire 30 will typically be drawn to a final finish diameter $D_{2S}$ between 20 $\mu$m and 250 $\mu$m.

The yield strength of wires 30 and 31, and thus their resilience, is influenced by the amount of strain-hardening deformation (e.g., cold work) applied to wires 30 and 31 to achieve the final diameter $D_{2S}$. In some cases, the yield strength may also be affected by a non-recrystallizing thermal treatment applied after the final drawing of the wire, as noted above. The ability to vary the strength and resilience of wires 30 and 31 allows use of the wire in resilient designs, such as for self-expanding stents, or for plastic-behaving designs, such as for balloon-expanding stents. At the same time, the alloying of an anti-pitting-corrosion element in the present material, N and optionally Cr and/or Mo as described herein, allows wire materials with retained cold work to retain the uniform degradation properties associated with Fe-Mn binary alloys, such that the wires are suitable for *in vivo* use.


Wire Biodegradation


In addition to material selection, the mechanical characteristics of wires 30 and 31 may be selected to determine its biodegradation rate. For example, the thicknesses of shell 32 and core 34 of composite wire 30 may be selected to control their respective biodegradation rates, with relatively thicker constructs requiring more time for biodegradation, and relatively thinner constructs requiring less time for biodegradation. Further, the geometry of the shell, core, and/or the overall formed device may result in certain regions of wire 30 being exposed to body tissue to a greater extent than other regions of wire 30, which may affect the biodegradation rates. Monolithic wire 31 may be similarly controlled for size and/or shape to promote faster or slower biodegradation.

Complex interactions between a stent and the surrounding biological and anatomic environment give rise to numerous application-specific, and sometimes patient-specific needs in stent wire mechanical properties. More particularly, stent reaction forces specific to the particular mechanical design of the constituent wires act upon the tunica intima of blood vessels. These reaction forces, arising from contact between the stent and the adjacent blood vessel, directly elicit a cellular response from the endothelium, thereby influencing the cell-blood interaction. Endothelial cells, in turn, regulate important biological responses such as vasodilation, gene expression, and inflammatory signaling sequences in response to mechanotransduction pathways associated with stimulation by wall-shearing blood flow. The endothelium also shields blood from pro-inflammatory cytokines and pro-aggregatory adhesion molecules found in the sub-intimal layers.

These processes depend upon complex signaling and feedback mechanisms which may be influenced by patient-specific factors including atherosclerotic disease, previous medical interventions, age and exercise. Advantageously, stents and wires made in accordance with the present disclosure offer the ability to optimize design to account for anatomy, blood and cell compatibility, long term endothelial functionality, fracture resistance, and patient-specific rates of bioabsorption. Such design optimization can be provided by, for example, cold work conditioning, thermomechanical processing, and material selection in accordance with the present disclosure. The particular effects of these variables on mechanical properties of the material and/or device are set forth in detail in the Examples below.

Wires and stents made in accordance with the present disclosure allow a surgeon to implant a naturally reactive stent over the long-term, thereby reducing late complications such as late-stent-thrombosis, relative vessel occlusion and lifelong anti-platelet therapy. When used in self-expanding, biocompatible, and biodegrading stent designs the present wire can further extend this ideal treatment option to the more-challenging vasculature of the extremities.

More specifically, bioabsorbable wires and stents made in accordance with the present disclosure can initially withstand flexion of mobile vessels of the extremities, give sufficient time for vessel remodeling, and then biodegrade. Thus, the present wire is ideally suited for use in stents implanted in high-flexion areas (i.e., extremities) and other demanding applications.

It is contemplated that balloon-expandable stent designs incorporating wire of the present disclosure will install with low balloon pressures and exert chronically lower expansion forces. Stent designs may also be tailored to bio-absorb after a desired time, such as after expected vessel remodeling, which can subsequently promote uninhibited endothelial function and vasoreactivity while also facilitating future reintervention as needed.

Wire made in accordance with the present disclosure can also be produced into stents which are specifically designed for long term therapy in young patients. Such designs may focus on the accommodation of somatic growth and the enablement of future reintervention. The ideal stent for CHD may be one that bio-absorbs at a desired, relatively slower rate in order to avoid vessel recoil before adequate remodeling has occurred.

The present wire also affords the opportunity for controllable degradation rates of stents to allow patient-dependent time for vessel remodeling. As noted above, patient-specific stent degradation rates also offer long-term benefit by allowing unimpeded reintervention and natural long term vasoreactivity.

For bimetallic composite wire 30, outer shell 32 may be designed as the relatively more slowly degrading component, such that overall degradation occurs at a relatively slower pace until the relatively fast-degrading core 34 begins to be exposed. At this stage, e.g. in the case of a wire construct 30 having an FeMnX outer shell 32 and a magnesium or magnesium alloy core, an electrochemical potential will drive the more rapid degradation of the core 34. In some designs, this intermediate degradation point may leave behind a thin FeMnX outer shell 32 which will possess reduced flexibility more similar to the vascular wall, thereby permitting more natural vessel movement and reactivity. Further, the remaining hollow outer shell 32 of FeMnX will present additional surface area to fluid contact *in vivo*, thereby causing the material to degrade more quickly than a comparable monolithic iron or iron alloy wire.

In wire constructs having an outer shell 32 formed of a more rapidly degrading material and a core 34 formed of a more slowly degrading material, the degradation process is expected to consume outer shell 32 and leave an intermediate and mostly continuous core 34. Similar to the embodiment described above, this relatively thin core element will provide improved flexibility, an increased rate of bioabsorption, and a concomitantly improved vessel healing response with a reduced risk of thrombosis, particle embolization, and restenosis compared to a monolithic bioabsorbable wire.

EXAMPLES

The following non-limiting Example illustrates various features and characteristics of the present invention, which is not to be construed as limited thereto.

Example 1 - Monolithic Fe-Mn-X Alloy Wire Materials

In this Example, exemplary monolithic Fe-Mn wires alloyed with N and optionally Cr, Mo or a combination thereof were produced, characterized and tested.

A Pitting Resistance Equivalent Number (PREN) can be used to compare the pitting corrosion resistance of various types of materials, based on their chemical compositions. In the present Example, PREN is calculated as:

$$PREN = Cr + 3.3(Mo + 0.5W) + 16N,$$

in which all factors are expressed as a wt.% quantity.

Table 1-1 summarizes the PREN for wires made in accordance with the present disclosure. As shown, nine wire samples were produced, with groups of three samples having varying levels of Cr, Mo and N used as alloying elements. Each group of three samples alloyed varying wt.% amounts of a given alloying element as shown.

Table 1-1

| Pitting Resistance Equivalent Number (PREN) for various Fe-Mn Alloy Wires Made in Accordance with the Present Disclosure | | | | | |
|---|---|---|---|---|---|
| Trial No. | Fe (wt.%) | Mn (wt.%) | Cr (wt.%) | Mo (wt.%) | N (wt.%) | PREN |
| | binary 65 | 35 | 0 | 0 | 0 | 0 |

(continued)

| Pitting Resistance Equivalent Number (PREN) for various Fe-Mn Alloy Wires Made in Accordance with the Present Disclosure | | | | | | |
|---|---|---|---|---|---|---|
| Trial No. | Fe (wt.%) | Mn (wt.%) | Cr (wt.%) | Mo (wt.%) | N (wt.%) | PREN |
| 1 | 64.8375 | 34.9125 | 0.25 | 0 | 0 | 0.25 |
| 2 | 64.675 | 34.825 | 0.5 | 0 | 0 | 0.5 |
| 7 | 65 | 34.95 | 0 | 0 | 0.05 | 0.8 |
| 4 | 65 | 34.75 | 0 | 0.25 | 0 | 0.825 |
| 3 | 64.35 | 34.65 | 1 | 0 | 0 | 1 |
| 8 | 65 | 34.85 | 0 | 0 | 0.15 | 2.4 |
| 5 | 65 | 34 | 0 | 1 | 0 | 3.3 |
| 9 | 65 | 34.55 | 0 | 0 | 0.45 | 7.2 |
| 6 | 65 | 32.5 | 0 | 2.5 | 0 | 8.25 |

Trials "binary" and 1-6 are not according to the invention.

As illustrated, PREN increases with corresponding increases in alloying elements. Thus, pitting corrosion kinetics can be expected to change for wires used in medical devices, such as stents, thereby facilitating a more uniform degradation in cold worked samples.

Example 2 - Monolithic Fe-Mn-X Alloy Wire Materials

In this Example, exemplary monolithic wires were produced, tested and characterized.

1. Production of Fe-Mn-X monolithic wires

Ingots were melted and cast into a 12.7 mm diameter by 150 mm length pre-form. Ingots with target chemistries in accordance with Table 1-1 were created by arc melting from primary metals of 99.95 wt.% minimum purity. These ingots were cold-formed into wire through conventional cold-working techniques including swaging and wire drawing combined with iterative annealing in order to restore ductility between respective cold-forming steps. All wires received a final recrystallization anneal treatment at a diameter of 0.64 mm prior to cold wire drawing to a finish diameter of 0.102 mm for testing and characterization.

2. Characterization of Fe-Mn-X monolithic wires

The rate of degradation of bioabsorbable materials can be measured in a laboratory using a simulated bodily environment, e.g. saline or buffered-saline supplemented with various mammalian serums or serum proteins. This testing modality presents an approximation of the true rate of degradation that would be observed in an implant subjected to *in vivo* conditions, and as such, is useful to compare the degradation of different materials and material conditions.

Variables which were expected to impact the degradation rate of materials include pH or relative acidity of the testing solution, protein adsorption and/or binding, *in vivo* immune response, fluid flow rate, local temperature, the local clearance rate of degradation byproducts and other complex variables such as stress-assisted corrosion, cellular adhesion, protein expression and fibrous encapsulation. When a solid foreign body is implanted, one of the first reactions to take place is protein adsorption; therefore, protein adsorption was expected to be an important variable in the initial degradation response. The quantity of protein adsorbed and the strength of the bond between the adsorbed proteins and the material are dependent upon the nature of the proteins and the chemical nature of the implant surface. In flowing human blood, two proteins that commonly adsorb to metallic and polymer surfaces include serum albumin and fibronectin. These proteins have been shown to adhere strongly to ferrous surfaces and this strong binding likely created a protective barrier which reduces the evacuation of the iron-hydroxide and iron oxide based degradation products, thereby retarding the degradation rate of the metal.

Wires were produced in accordance with the material specifications of Table 1-1: "binary" (i.e., Fe-Mn wire without an anti-pitting corrosion element, used as a control), and the wire alloys of trial nos. 2, 5, 6 and 8 of Table 1-1. These test materials were formed into 1.25 mm diameter single wire coil stents and tested for degradation rate and for the occurrence of fracture by subjecting them to low speed stirring in a 37 ± 1 °C adult bovine serum environment with once per week serum changes. At each weekly serum change, samples were examined for mass loss by precision weighing of cleaned and dried samples. Samples were also examined for fracture and the number of fractures was recorded according to the number of fully separated pieces contained in the sample vial.

Table 2-1 summarizes the number of fractures observed for above-described wire compositions (i.e., those listed "binary" and as trial nos. 2, 5, 6 and 8 of Table 1-1). As illustrated in Table 2-1, after 50 days of incubation, alloy trial nos. 2 and 8 were observed to contain a mean of 1 and 1.75 fractures per stent, the lowest number of fracture observed for the group. A mean of 5 fractures were observed for the binary Fe-Mn alloy over 50 days of testing.

Table 2-1

Mean number of breaks for N = 4 over given time (days)

| Time | (days) | → | | | | | | | |
|---|---|---|---|---|---|---|---|---|---|
| **Alloy** 0 | 8 | 15 | 21 | 22 | 24 | 29 | 36 | 43 | 50 |
| **2** 0.00 | 0.00 | 0.00 | 0.00 | 0.00 | 0.00 | 0.00 | 0.50 | 0.50 | 1.00 |
| **5** 0.00 | 0.00 | 0.00 | 0.00 | 0.00 | 0.00 | 2.50 | 2.50 | 2.50 | 3.75 |
| **6** 0.00 | 1.25 | 2.00 | 2.00 | 2.00 | 2.50 | 3.50 | 4.25 | 5.25 | 6.50 |
| **8** 0.00 | 0.00 | 0.00 | 0.00 | 0.00 | 0.00 | 0.50 | 0.50 | 0.50 | 1.75 |
| **Binary** 0.00 | 0.00 | 0.00 | 0.50 | 1.00 | 1.00 | 2.25 | 3.50 | 4.25 | 5.00 |

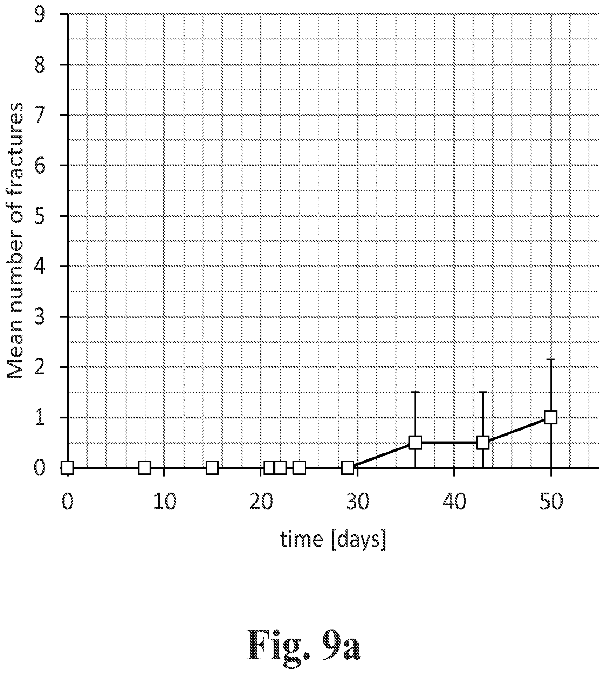

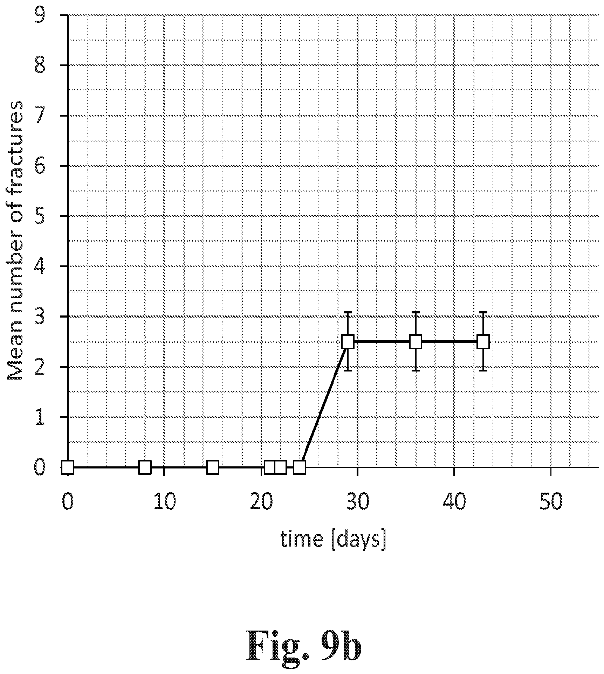

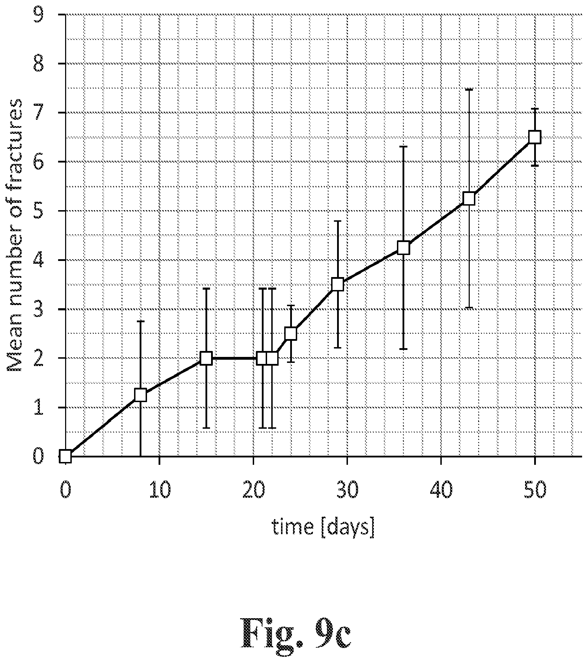

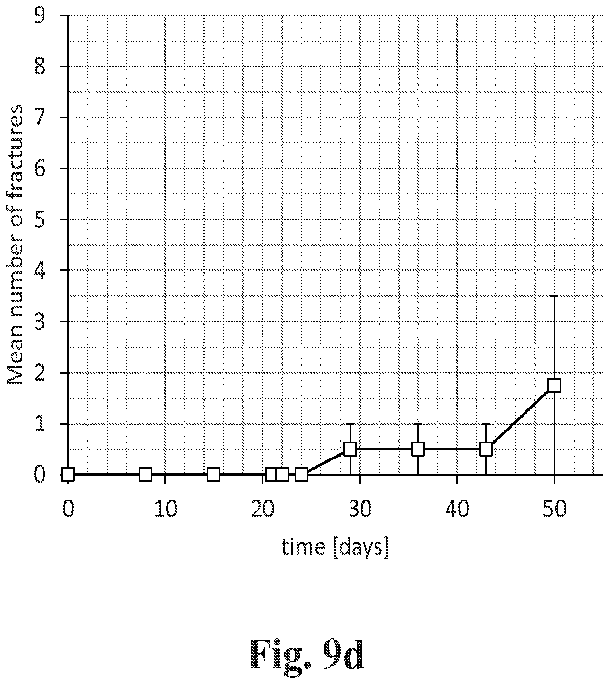

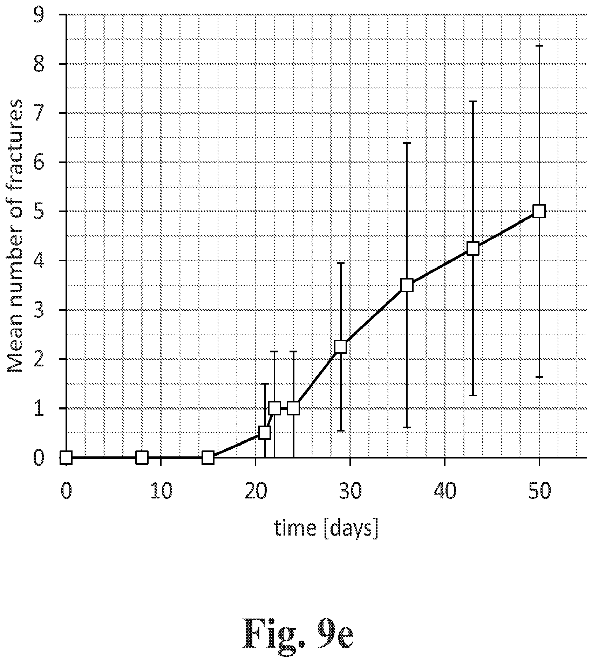

The number of fractures for each tested sample are presented in Figs. 9a-9e. In particular, Fig. 9a illustrates the data for alloy no. 2; Fig. 9b illustrates the data for alloy no. 5; Fig. 9c illustrates the data for alloy no. 6; Fig. 9d illustrates the data for alloy no. 8; and Fig. 9e illustrates the data for the control "Binary" alloy.

Table 2-2 illustrates the standard deviation calculated for the data presented in Table 2-1. These standard deviations are graphically illustrated as vertical bars for each time entry in Figs. 9a-9e respectively.

Table 2-2

Standard deviation of number of breaks for N = 4 over given time (days)

| Time | (days) | → | | | | | | | |
|---|---|---|---|---|---|---|---|---|---|
| **Alloy** 0 | 8 | 15 | 21 | 22 | 24 | 29 | 36 | 43 | 50 |
| **2** 0.00 | 0.00 | 0.00 | 0.00 | 0.00 | 0.00 | 0.00 | 1.00 | 1.00 | 1.15 |
| **5** 0.00 | 0.00 | 0.00 | 0.00 | 0.00 | 0.00 | 0.58 | 0.58 | 0.58 | 1.71 |
| **6** 0.00 | 1.50 | 1.41 | 1.41 | 1.41 | 0.58 | 1.29 | 2.06 | 2.22 | 0.58 |
| **8** 0.00 | 0.00 | 0.00 | 0.00 | 0.00 | 0.00 | 1.00 | 1.00 | 1.00 | 2.06 |
| **Binary** 0.00 | 0.00 | 0.00 | 1.00 | 1.15 | 1.15 | 1.71 | 2.89 | 2.99 | 3.37 |

Example 3 - Additional Candidate Materials for DFT Constructions

In this Example, exemplary bimetal composite wires and high strength iron monolith wires were produced, tested and characterized. In addition, three benchmark alloy wires were produced, tested and characterized for comparison to the exemplary wires.

1. Production of bimetal composite and monolithic wires

For Wire # 1-3 in Tables 3-1 and 3-4 below, a pure Fe rod of dimension F mm outside diameter (OD) was processed as monolithic (solid) wire.

Similarly, for Wire # 7-9 in Tables 3-3 and 3-4 below, 316L stainless steel, MP35N and Nitinol alloy wire respectively, of dimension H mm outside diameter (OD) were processed as monolithic wire.

For Wire # 4-6 in Tables 3-2 and 3-4 below, a pure Fe tube of dimension A mm outside diameter (OD) x dimension B mm inside diameter (ID) was filled and drawn down over dimension C mm outside diameter (OD) pure Mg rod to create a first composite having the area fraction specified. Value D is the area fraction as defined by the ratio of the core area to overall wire area.

All wires were repetitively drawn and annealed, with appropriate levels of strain hardening to impart relatively high strength to the resulting wire product, to a final nominal finish OD of 125 um, and the wires were spooled. The tensile strength properties of the wires were measured in a uniaxial tensile test on an Instron Model 5565 test machine at 24°C in ambient shop air.

Table 3-1

| Exemplary monolithic wires | | | |
|---|---|---|---|
| Wire # | Starting Size, mm (F) | Finish Diameter, mm (G) | Material Designation |
| 1 | 12.7 | 0.127 | Fe-50 |
| 2 | 12.7 | 0.127 | Fe-90 |
| 3 | 12.7 | 0.127 | Fe-99 |

Table 3-2

| Exemplary DFT wires | | | | | |
|---|---|---|---|---|---|
| Wire # | Tube OD, mm (A) | Tube ID, mm (B) | Core ID, mm (C) | Core Ratio, % (D) | Finish Diameter, mm (E) | Material Designation |
| 4 | 10 | 7 | 3.2 | 25 | 0.127 | Fe-DFT-25%Mg |
| 5 | 5 | 4 | 3.2 | 25 | 0.127 | Fe-DFT-57%Mg |
| 6 | 11.5 | 5.8 | 5.4 | 25 | 0.127 | Fe35Mn-DFT-25%MgZM21 |

Table 3-3

| Monolithic benchmark alloy wires | | | |
|---|---|---|---|
| Wire # | Starting Size, mm (H) | Finish Diameter, mm (I) | Material Designation |
| 7 | 2.5 | 0.127 | 316L |
| 8 | 1.6 | 0.127 | MP35N |
| 9 | 2.1 | 0.127 | Nitinol |

The tensile strength properties observed are similar to those of known materials such as Co-Ni-Cr/Tantalum composite wires used in stent designs and therefore are expected to be suitable for subsequent forming and wall support functionality.

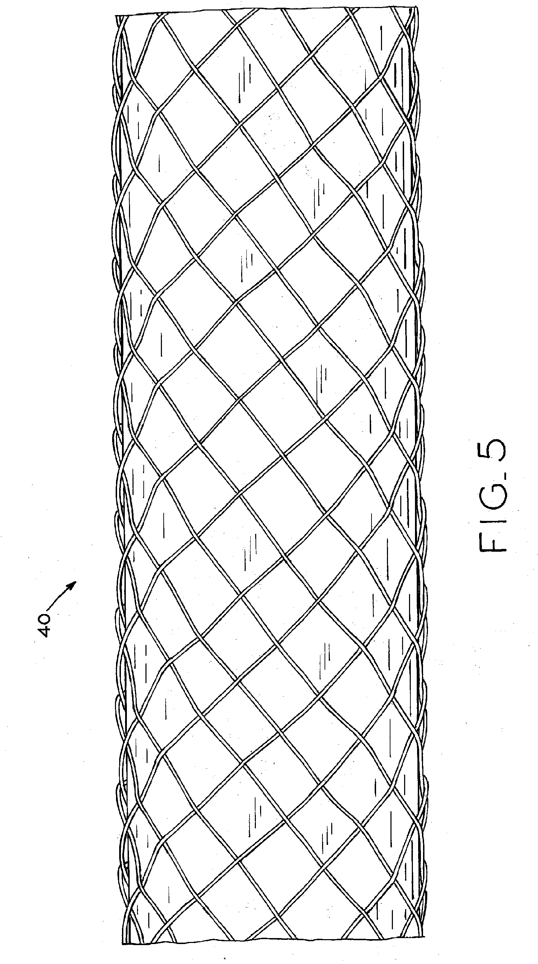

Several hundred meter lengths of the wires were also successfully braided into a 24 wire count, 90° braid angle, Ø5 mm ID tubular stent structures shown in Fig. 5, suitable for use as an arterial support structure similar to the stent shown in Fig. 1A.

2. Characterization of mechanical properties in tension, flexure and cyclic loading

In this Example, mechanical testing is performed on exemplary bimetal composite and monolithic wires, and mechanical properties of the wires are characterized.

In order to maximize therapeutic benefit, any vessel scaffold should be able to mechanically withstand both the static and pulsatile dynamic radial forces exerted by the wall after implantation. The static strength of the scaffold will ideally be sufficient to prevent acute recoil and negative remodeling for at least 3-6 months after implantation. Pulsatile loading associated with the beating heart will impart $10^7$ load cycles during this period of remodeling. In order to ensure suitable material strength, material systems are tested and compared against materials which are used routinely in clinical practice such as 316L stainless steel, cobalt-chrome-moly alloy (CoNiCr, or MP35N®) and Nitinol shape memory alloy (NiTi). The aim of testing is to benchmark these alloys via uniaxial tension and durability against cyclic flexural fatigue damage.

a. Tensile Strength

The composite tensile strength and toughness of exemplary wires in accordance with the present disclosure is expected to increase as a function of the fractional Fe-constituency.

i. Experimental Technique: Tensile Testing

Destructive uniaxial tension testing of the wire materials is used to quantify the ultimate strength, yield strength, axial stiffness and ductility of candidate materials, using methods described in Structure-Property Relationships in Conventional and Nanocrystalline NiTi Intermetallic Alloy Wire, Journal of Materials Engineering and Performance 18, 582-587 (2009) by Jeremy E. Schaffer. These tests are run using servo-controlled Instron load frames in accordance with industry standards for the tension testing of metallic materials.

Bioabsorbable and benchmark alloy wires are destructively tested in a monotonic, single tensile load-increasing cycle, at 25°C at a strain rate of $10^{-3}$ s$^{-1}$ using an Instron Model 5500 series load frame (Instron, Norwood, MA, USA).

ii. Results

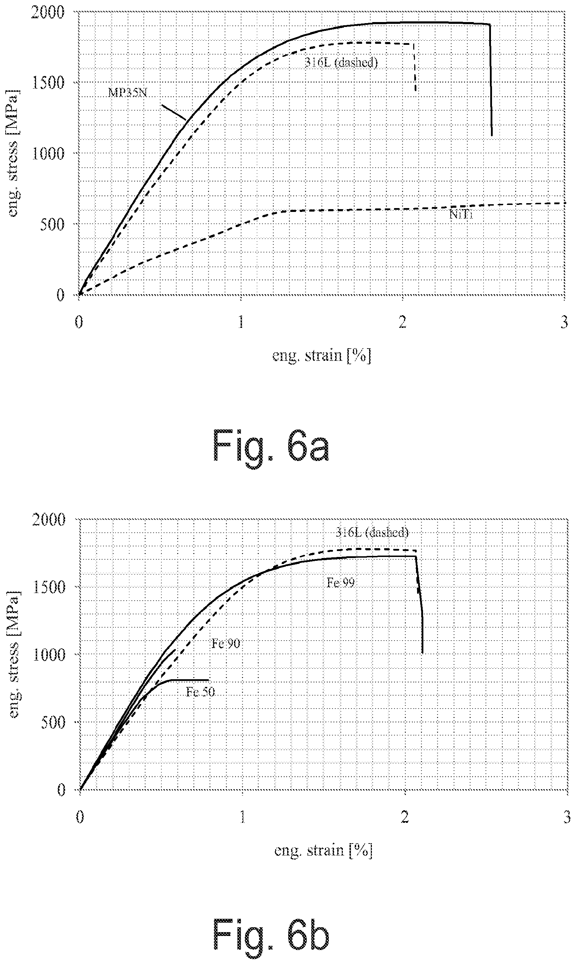

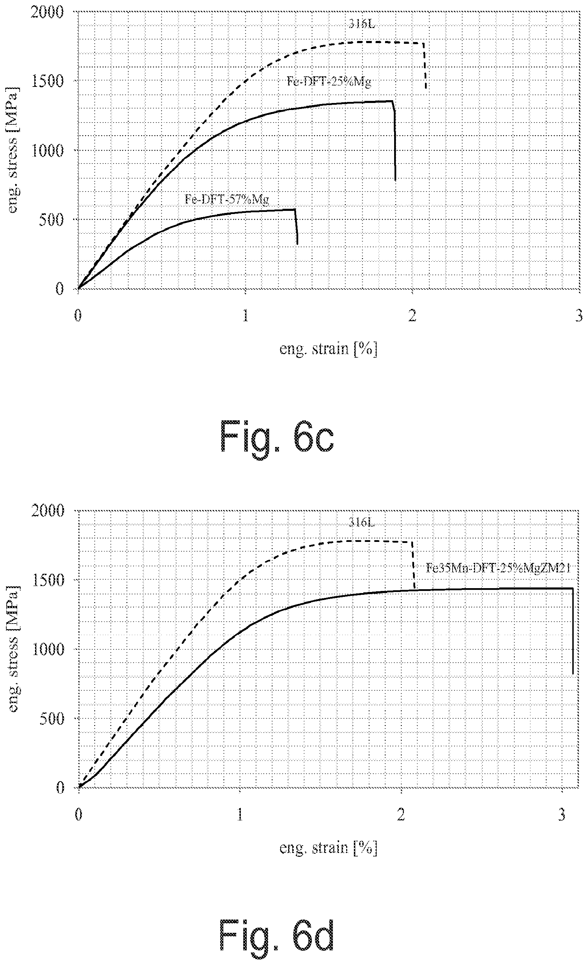

Figures 6a-6d are plots of stress-strain data for individual 125 $\mu$m wire.

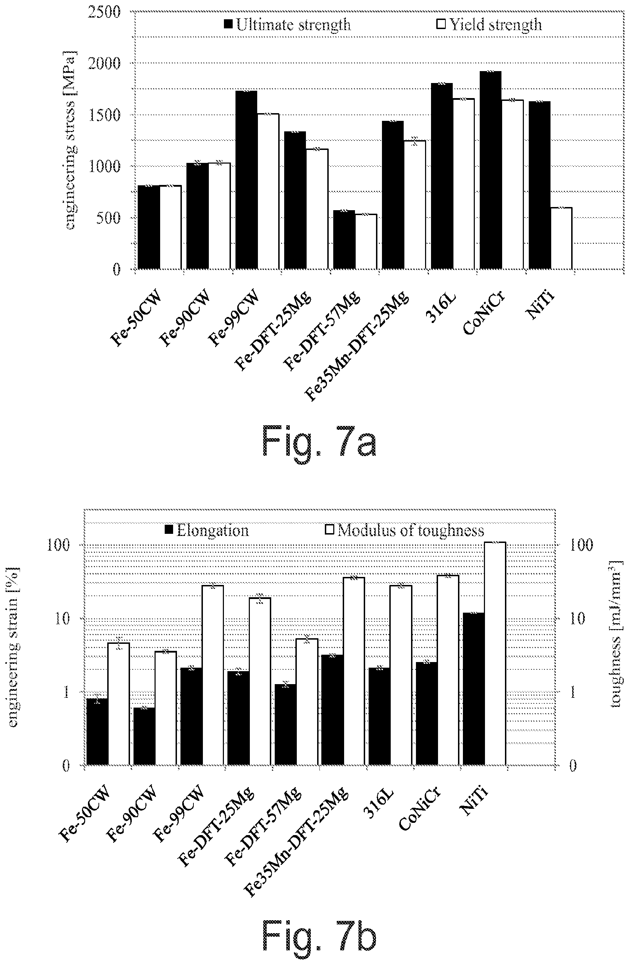

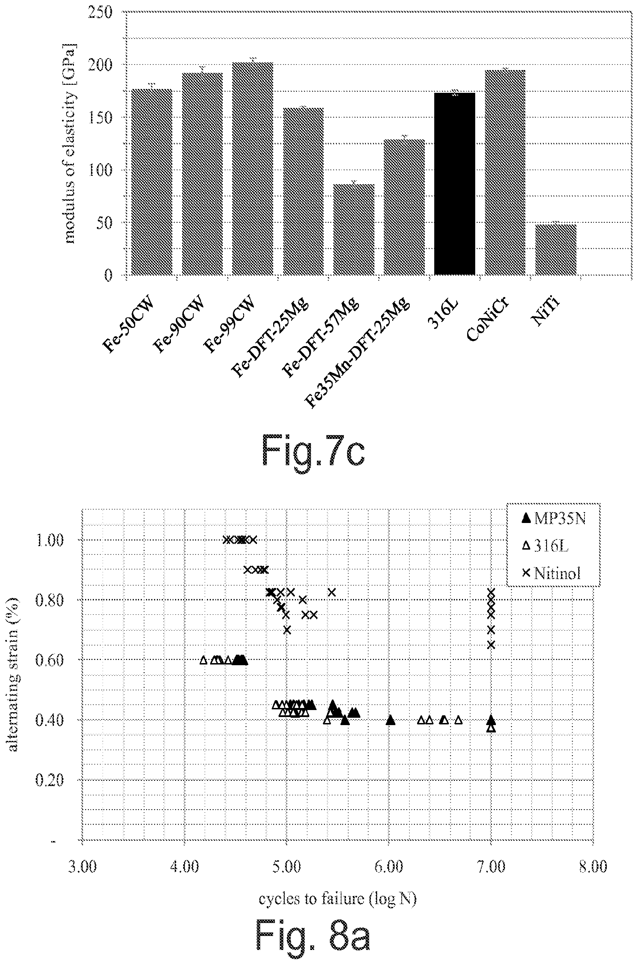

Ultimate tensile strength, yield strength, elongation at rupture, modulus of elasticity and modulus of toughness were calculated from similar plots for each sample tested at six breaks per sample (N=6). The results of such testing are summarized in Figures 7a-7c, in which illustrated error bars are equivalent to one standard deviation, and presented in Table 3-4 below. Table 3-4 includes numerical values for the data presented graphically in Figs. 7a-7c, with the nominal value of one standard deviation in parentheses below each respective tabular value.

Table 3-4

| Mechanical Property Data For Nominally 125$\mu$m ($\varnothing$.005") Bimetal Composite, Iron Monolith and Benchmark Wires. | | | | | | |
|---|---|---|---|---|---|---|
| Wire # - Material | Ultimate strength $S_U$ [MPa] | Yield strength $S_Y$ [MPa] | Elongation $e_R$ [%] | Young's Modulus of Elasticity E [GPa] | Modulus of Toughness $E_T$ [mJ/mm$^3$] | Resilience $S_Y$/ E [%] |
| 1 - Fe-50 | 813 | 810 | 0.809 | 177 | 4.64 | 0.46 |
| | (2.36) | (2.21) | (0.110) | (5.4) | (0.82) | (0.0013) |
| 2 - Fe-90 | 1032 | 1032 | 0.609 | 192 | 3.52 | 0.54 |
| | (18.9) | (18.9) | (0.022) | (6.0) | (0.21) | (0.0102) |
| 3 - Fe-99 | 1728 | 1505 | 2.12 | 202 | 27.9 | 0.75 |
| | (0.79) | (5.73) | (0.130) | (4.0) | (2.3) | (0.0029) |
| 4 - Fe-DFT-25%Mg | 1332 | 1163 | 1.90 | 159 | 18.8 | 0.73 |
| | (4.25) | (10.1) | (0.189) | (1.7) | (-2.5) | (0.0065) |
| 5 - Fe-DFT-57%Mg | 570 | 532 | 1.27 | 86 | 5.23 | 0.62 |
| | (2.47) | (4.34) | (0.107) | (2.9) | (054) | (0.0052) |
| 6 - Fe35Mn-25%MgZM21 | 1439 | 1243 | 3.16 | 129 | 35.8 | 0.97 |
| | (2.66) | (36.2) | (0.142) | (4.0) | (1.6) | (0.0290) |
| 7 - 316L | 1801 | 1650 | 2.13 | 173 | 27.9 | 0.95 |
| | (1.76) | (7.81) | (0.100) | (2.4) | (1.8) | (0.0046) |
| 8 - MP35N® | 1922 | 1640 | 2.56 | 194 | 38.1 | 0.84 |
| | (2.45) | (9.89) | (0.109) | (2.1) | (2.0) | (0.0051) |
| 9 - Nitinol | 1626 | 596 | 11.9 | 48 | 108 | 1.25 |
| | (1.34) | (3.48) | (0.088) | (2.8) | (1.6) | (0.0077) |

As set forth in Table 3-4, tensile test data show that the mechanical properties of the exemplary monolithic wires are comparable and/or statistically similar to the benchmark non-biodegradable materials including 316L stainless steel wire.

The ultimate tensile strength and stiffness of the exemplary bimetal composite wires were intermediate, falling below 316L. The modulus of toughness of the Fe-DFT-25%Mg and Fe35Mn-25%MgZM21 composites, at 18.8 mJ/mm$^3$ and 35.8 mJ/mm$^3$ respectively, were similar to that of benchmark 316L and MP35N (27.9 mJ/mm$^3$ and 38.1 mJ/mm$^3$, respectively).

b. Fatigue Resistance

The mechanical fatigue durability of *in vivo* wires made in accordance with the present disclosure can be expected to improve by compositing with Fe or Fe alloy through enhanced surface resistance to plastic deformation in the cold work (CW) strain-hardened Fe exterior.

i. Experimental Technique: Rotary beam fatigue testing

Candidate material durability was empirically determined by testing to failure under cyclic load conditions using rotary beam fatigue testing methods as described in Structure-Property Relationships in Conventional and Nanocrystalline NiTi Intermetallic Alloy Wire, incorporated by reference above. These tests are run using A/C synchronous motor-driven, rotary, load frames achieving a cyclic rate of 60 Hz. The tests are initially run in ambient lab air at 24 $\pm$ 3°C at a load ratio, defined as the ratio of the minimum to maximum load strain, of R = - 1 to rupture or cessation of testing at $10^7$ cycles. From these data, namely the number of cycles to failure and load strains, Woehler load-life diagrams are computed and compared against the benchmark alloy systems.

125 $\mu$m bioabsorbable and benchmark alloy samples are loaded into a motor driven pin vise and elastically loaded to a geometrically defined strain level according to:

$$\varepsilon_{amp} = d \,/\, (d + D),$$

where the strain amplitude ($\varepsilon_{amp}$) is defined by the wire diameter, *d*, and the bend diameter, D. Samples are rotated at 3600 rpm in ambient air (T = 23 $\pm$ 3°C) until rupture is achieved or until test termination at $10^7$ cycles.

ii. Results

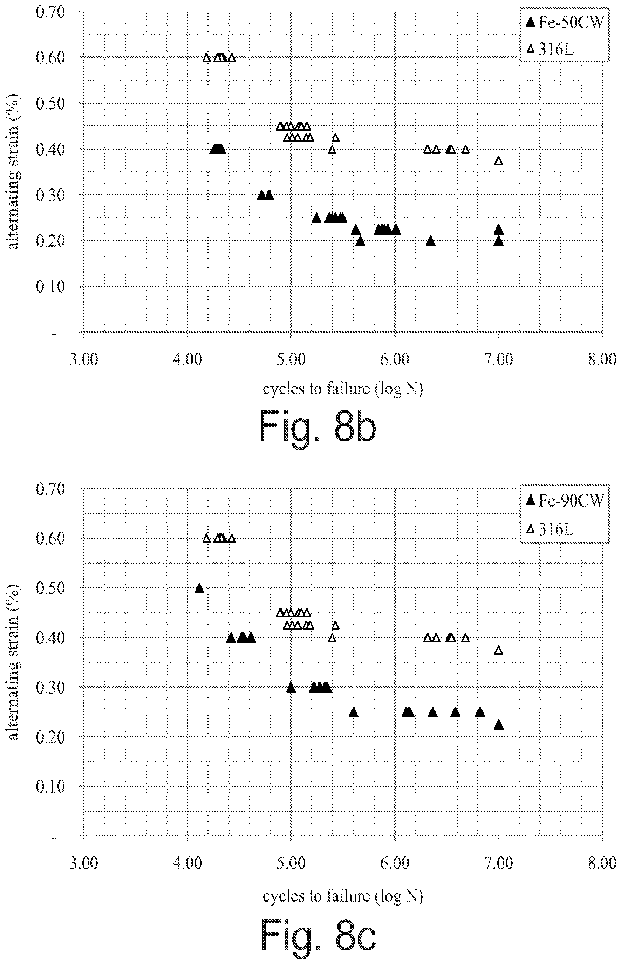

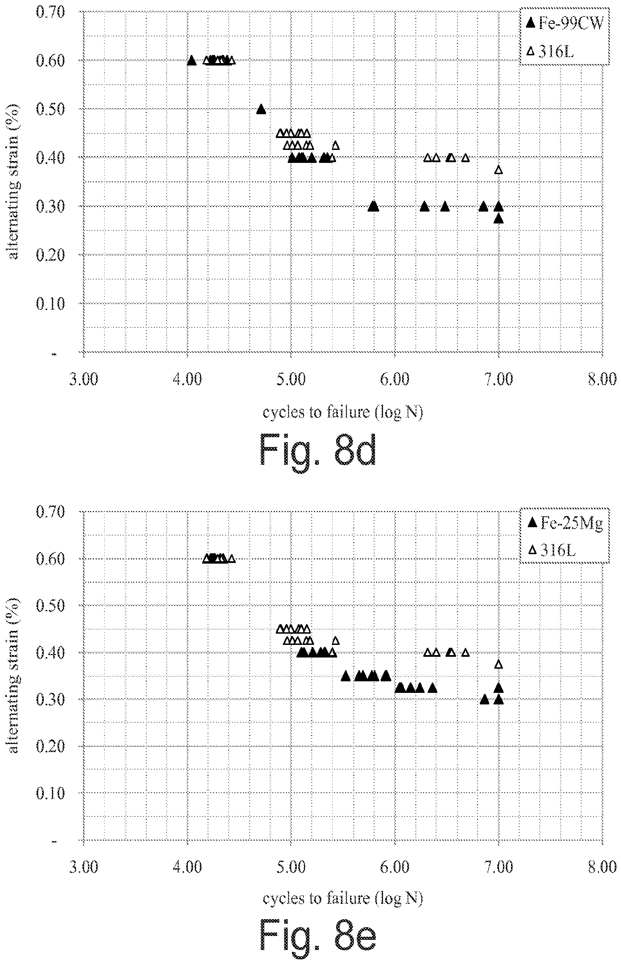

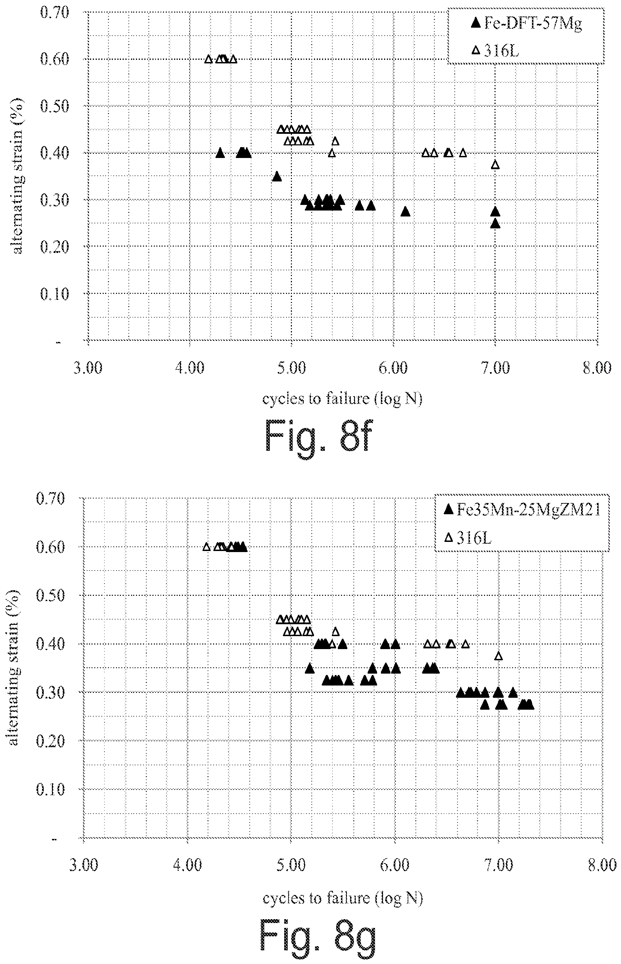

Figures 8a-8g show a summary of the results plotted in a strain amplitude-life diagram. Figure 8a shows results for three benchmark monolithic wires. Figures 8b-8d show results for exemplary monolithic wires prepared in accordance with the present disclosure, as compared to the benchmark 316L stainless material. Figures 8e-8g show results for exemplary bimetal composite wires prepared in accordance with the present disclosure, as compared to the benchmark 316L stainless material.

At $10^7$ cycles (i.e., "log 7.00" cycles in the nomenclature of Figs. 8a-8g), the fatigue strength the exemplary monolithic and bimetal composite materials (i.e., from about 0.2% strain to above 0.3% strain) was comparable to 316L (0.37% strain).

Commercially available 316L BX stents utilize relatively soft-annealed metal, with alternating strain limits at $10^7$ cycles of approximately 0.2%, similar to the lowest fatigue strength found for the exemplary materials for iron with 50% strain hardening (or "CW" meaning "cold work").

**Claims**

1. A medical device comprising:

   a monolithic wire having stored deformation energy from cold work such that a retained cold work ratio of grain length to grain width is between 10:1 and 50:1, the wire comprising between 45 wt.% and 85 wt.% iron (Fe), between 15 wt.% and 55 wt.% manganese (Mn), and an anti-corrosive alloying element comprising between 0.01 wt.% and 0.45 wt.% nitrogen (N),
   the wire consisting entirely of biodegradable material such that the wire is able to be chemically broken down in a physiological environment.

2. The medical device of claim 1, wherein said monolithic wire further comprises at least one of:

   between 0.05 wt.% and 1.3 wt.% chromium (Cr); and
   between 0.10 wt.% and 5.0 wt.% molybdenum (Mo).

3. The medical device of claim 1, wherein said monolithic wire comprises a wire having a round cross-section and a diameter less than 1 mm.

4. The medical device of claim 1, wherein said monolithic wire includes retained cold work such that respective individual grains throughout said monolithic wire are elongated to define a ratio of grain length to grain width of at least 15:1.

5. The medical device of claim 1, wherein said monolithic wire comprises chromium (Cr) in an amount between 0.25 wt.% and 0.7 wt.%.

6. The medical device of claim 1, wherein said monolithic wire comprises molybdenum (Mo) in an amount between 0.50 wt.% and 2.0 wt.%.

7. The medical device of claim 1, wherein said monolithic wire comprises nitrogen (N) in an amount between 0.05 wt.% and 0.12 wt.%.

8. A stent comprising the wire material of claim 1.

9. A method of manufacturing a biodegradable wire (31), comprising the steps of:
   providing a wire made of Fe-Mn-X alloy, the wire comprising:

   iron (Fe) in the amount of at least 61 wt.%;
   manganese (Mn) in the amount of at least 31 wt.% manganese (Mn); and
   a quantity of an anti-corrosive alloying element (X) comprising:
   nitrogen (N) in an amount between 0.01 wt.% and 0.45 wt.%, and
   strengthening the wire by imparting cold work at room temperature to the wire until a retained cold work ratio of grain length to grain width in the Fe-Mn-X alloy is between 10:1 and 50:1.

10. The method of Claim 9, wherein said step of imparting cold work comprises drawing the wire construct from a first outer diameter to a second outer diameter less than the first outer diameter.

11. The method of Claim 9, wherein said step of providing a wire comprises:

    providing an outer shell made of a first biodegradable material;
    inserting a core into the outer shell to form a wire construct, the core formed of a second biodegradable material, one of said first and second biodegradable metallic materials comprising said Fe-Mn-X alloy material, and the other of said first and second biodegradable metallic materials comprising a second material different from said Fe-Mn-X alloy.

12. The method of Claim 11 wherein said outer shell is made of said Fe-Mn-X alloy material and said core is selected from the group consisting of pure magnesium (Mg) and a magnesium-based alloy (Mg alloy).

13. The method of claim 9, further comprising the additional step of forming the wire into a stent.

14. The method of Claim 9, further comprising, after said imparting step, the additional step of annealing the wire construct by heat treatment at a temperature low enough to prevent recrystallization of the material.


**Patentansprüche**

1. Medizinische Vorrichtung, umfassend:

   einen monolithischen Draht, auf dem Verformungsenergie aus einer Kaltumformung gespeichert ist, sodass ein bewahrtes Kaltumformungsverhältnis der Kornlänge zur Kornbreite zwischen 10:1 und 50:1 liegt, wobei der

**EP 2 872 663 B1**

Draht zwischen 45 Gew.-% und 85 Gew.-% Eisen (Fe), zwischen 15 Gew.-% und 55 Gew.-% Mangan (Mn) und ein Legierungselement zum Korrosionsschutz umfasst, umfassend

zwischen 0,01 Gew.-% und 0,45 Gew.-% Stickstoff (N),

wobei der Draht vollständig aus einem biologisch abbaubaren Material besteht, sodass der Draht dazu in der Lage ist, in einer physiologischen Umgebung chemisch aufgeschlossen zu werden.

2. Medizinische Vorrichtung nach Anspruch 1, wobei der monolithische Draht ferner mindestens eines der Folgenden umfasst:

    zwischen 0,05 Gew.-% und 1,3 Gew.-% Chrom (Cr); und
    zwischen 0,10 Gew.-% und 5,0 Gew.-% Molybdän (Mo).

3. Medizinische Vorrichtung nach Anspruch 1, wobei der monolithische Draht einen Draht umfasst, der einen runden Querschnitt und einen Durchmesser aufweist, der kleiner als 1 mm ist.

4. Medizinische Vorrichtung nach Anspruch 1, wobei der monolithische Draht eine bewahrte Kaltumformung einschließt, sodass entsprechende einzelne Körner in dem monolithischen Draht verlängert werden, um ein Verhältnis der Kornlänge zur Kornbreite von mindestens 15:1 zu definieren.

5. Medizinische Vorrichtung nach Anspruch 1, wobei der monolithische Draht Chrom (Cr) in einer Menge zwischen 0,25 Gew.-% und 0,7 Gew.-% umfasst.

6. Medizinische Vorrichtung nach Anspruch 1, wobei der monolithische Draht Molybdän (Mo) in einer Menge zwischen 0,50 Gew.-% und 2,0 Gew.-% umfasst.

7. Medizinische Vorrichtung nach Anspruch 1, wobei der monolithische Draht Stickstoff (N) in einer Menge zwischen 0,05 Gew.-% und 0,12 Gew.-% umfasst.

8. Stent, umfassend das Drahtmaterial nach Anspruch 1.

9. Verfahren zur Herstellung eines biologisch abbaubaren Drahts (31), umfassend die folgenden Schritte:

    Bereitstellen eines Drahts, der aus einer Fe-Mn-X-Legierung hergestellt wird,
    wobei der Draht Folgendes umfasst:

        Eisen (Fe) in der Menge von mindestens 61 Gew.-%;
        Mangan (Mn) in der Menge von mindestens 31 Gew.-% Mangan (Mn); und
        eine Menge eines Legierungselements (X) zum Korrosionsschutz, umfassend:

    Stickstoff (N) in einer Menge zwischen 0,01 Gew.-% und 0,45 Gew.-%, und Verstärken des Drahts, indem eine Kaltumformung an dem Draht bei Raumtemperatur ausgeführt wird, bis ein bewahrtes Kaltumformungsverhältnis der Kornlänge zur Kornbreite in der Fe-Mn-X-Legierung zwischen 10:1 und 50:1 liegt.

10. Verfahren nach Anspruch 9, wobei der Schritt des Ausführens einer Kaltumformung das Ziehen des Drahtkonstrukts von einem ersten Außendurchmesser zu einem zweiten Außendurchmesser umfasst, der kleiner als der erste Außendurchmesser ist.

11. Verfahren nach Anspruch 9, wobei der Schritt des Bereitstellens eines Drahts Folgendes umfasst:

    Bereitstellen einer Außenhülle, die aus einem ersten biologisch abbaubaren Material besteht;
    Einsetzen eines Kerns in die Außenhülle, um ein Drahtkonstrukt zu bilden, wobei der Kern aus einem zweiten biologisch abbaubaren Material gebildet wird, eines des ersten und zweiten biologisch abbaubaren Metallmaterials, umfassend das Fe-Mn-X-Legierungsmaterial, und das andere des ersten und zweiten biologisch abbaubaren Metallmaterials, umfassend ein zweites Material, das sich von der Fe-Mn-X-Legierung unterscheidet.

12. Verfahren nach Anspruch 11, wobei die Außenhülle aus dem Fe-Mn-X-Legierungsmaterial besteht und der Kern aus der Gruppe ausgewählt ist, bestehend aus reinem Magnesium (Mg) und einer Legierung auf Magnesiumbasis (Mg-Legierung).

18

**13.** Verfahren nach Anspruch 9, ferner umfassend den zusätzlichen Schritt des Formens des Drahts zu einem Stent.

**14.** Verfahren nach Anspruch 9, ferner umfassend, nach dem Ausführungsschritt, den zusätzlichen Schritt des Glühens des Drahtkonstrukts durch eine Wärmebehandlung bei einer Temperatur, die niedrig genug ist, um eine Rekristallisation des Materials zu verhindern.

**Revendications**

**1.** Dispositif médical comprenant :

un fil monolithique ayant stocké une énergie de déformation provenant d'un écrouissage de telle sorte qu'un rapport d'écrouissage retenu entre longueur de grain et largeur de grain est entre 10 : 1 et 50 : 1, le fil comprenant entre 45 % en poids et 85 % en poids de fer (Fe), entre 15 % en poids et 55 % en poids de manganèse (Mn), et un élément d'alliage anticorrosif comprenant entre 0,01 % en poids et 0,45 % en poids d'azote (N), le fil étant entièrement constitué de matériau biodégradable de telle sorte que le fil est capable de se désintégrer chimiquement dans un environnement physiologique.

**2.** Dispositif médical selon la revendication 1, dans lequel ledit fil monolithique comprend en outre au moins l'un parmi :

entre 0,05 % en poids et 1,3 % en poids de chrome (Cr) ; et
entre 0,10 % en poids et 5,0 % en poids de molybdène (Mo).

**3.** Dispositif médical selon la revendication 1, dans lequel ledit fil monolithique comprend un fil ayant une section transversale ronde et un diamètre inférieur à 1 mm.

**4.** Dispositif médical selon la revendication 1, dans lequel ledit fil monolithique comporte un écrouissage retenu de telle sorte que des grains individuels respectifs tout le long dudit fil monolithique sont allongés pour définir un rapport entre longueur de grain et largeur de grain d'au moins 15 : 1.

**5.** Dispositif médical selon la revendication 1, dans lequel ledit fil monolithique comprend du chrome (Cr) dans une quantité entre 0,25 % en poids et 0,7 % en poids.

**6.** Dispositif médical selon la revendication 1, dans lequel ledit fil monolithique comprend du molybdène (Mo) dans une quantité entre 0,50 % en poids et 2,0 % en poids.

**7.** Dispositif médical selon la revendication 1, dans lequel ledit fil monolithique comprend de l'azote (N) dans une quantité entre 0,05 % en poids et 0,12 % en poids.

**8.** Endoprothèse comprenant le matériau de fil de la revendication 1.

**9.** Procédé de fabrication d'un fil biodégradable (31), comprenant les étapes de :
fourniture d'un fil réalisé en alliage de Fe-Mn-X, le fil comprenant :

du fer (Fe) dans la quantité d'au moins 61 % en poids ;
du manganèse (Mn) dans la quantité d'au moins 31 % en poids de manganèse (Mn) ; et
une quantité d'un élément d'alliage anticorrosif (X) comprenant :
de l'azote (N) dans une quantité entre 0,01 % en poids et 0,45 % en poids, et renforcement du fil en transmettant l'écrouissage à température ambiante au fil jusqu'à ce qu'un rapport d'écrouissage retenu entre longueur de grain et largeur de grain dans l'alliage de Fe-Mn-X soit entre 10 : 1 et 50 : 1.

**10.** Procédé selon la revendication 9, dans lequel ladite étape de transmission d'un écrouissage comprend l'étirage du produit de construction de fil d'un premier diamètre extérieur à un second diamètre extérieur inférieur au premier diamètre extérieur.

**11.** Procédé selon la revendication 9, dans lequel ladite étape de fourniture d'un fil comprend :

la fourniture d'une enveloppe extérieure réalisée en un premier matériau biodégradable ;

l'insertion d'une âme dans l'enveloppe extérieure pour former un produit de construction de fil, l'âme étant formée d'un second matériau biodégradable,

l'un desdits premier et second matériaux métalliques biodégradables comprenant ledit matériau d'alliage de Fe-Mn-X, et l'autre desdits premier et second matériaux métalliques biodégradables comprenant un second matériau différent dudit alliage de Fe-Mn-X.

12. Procédé selon la revendication 11, dans lequel ladite enveloppe extérieure est réalisée en ledit matériau d'alliage de Fe-Mn-X et ladite âme est choisie dans le groupe constitué de magnésium pur (Mg) et d'un alliage à base de magnésium (alliage de Mg).

13. Procédé selon la revendication 9, comprenant en outre l'étape supplémentaire de formation du fil en une endoprothèse.

14. Procédé selon la revendication 9, comprenant en outre, après ladite étape de transmission, l'étape supplémentaire de recuit du produit de construction de fil par traitement thermique à une température suffisamment basse pour empêcher une recristallisation du matériau.

FIG. 1A

FIG. 1B

FIG. 2

FIG. 3a

FIG. 3b

12    12    12    30,31

D1S

G1    FIG. 3c

14    14    14    30,31

G3    D2S

G2    FIG. 3d

FIG. 4a

FIG. 4b

FIG. 4c

FIG. 4d

EP 2 872 663 B1

EP 2 872 663 B1

40

FIG. 5

**Fig. 6a**

**Fig. 6b**

Fig. 6c

Fig. 6d

Fig. 7a

Fig. 7b

Fig.7c

Fig. 8a

Fig. 8b

Fig. 8c

Fig. 8d

Fig. 8e

Fig. 8f

Fig. 8g

**Fig. 9a**

Fig. 9b

Fig. 9c

Fig. 9d

Fig. 9e

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009198320 A1
- US 39509009
- US 20090260852 A

**Non-patent literature cited in the description**

- **JEREMY E. SCHAFFER.** Structure-Property Relationships in Conventional and Nanocrystalline NiTi Intermetallic Alloy Wire. *Journal of Materials Engineering and Performance,* 2009, vol. 18, 582-587
- *Structure-Property Relationships in Conventional and Nanocrystalline NiTi Intermetallic Alloy Wire*